# EUROPEAN PATENT APPLICATION

(11) **EP 4 643 885 A1**
(43) Date of publication of application: **05.11.2025**
(21) Application number: 23910375.7
(22) Date of filing: 22.12.2023
(51) Int. Cl.: A61K 47/68, C07D 491/22, A61P 35/00

(54) **ANTIBODY-DRUG CONJUGATE FROM N-OXYCYCLOALKYL SUBSTITUTED CAMPTOTHECIN DERIVATIVE**

(30) Priority: 29.12.2022 CN 202211743667
(71) Applicant: Hangzhou Adcoris Biopharma Co., Ltd, Hangzhou, Zhejiang 310056 (CN)
(72) Inventor: HUANG, Yunsheng, Hangzhou, Zhejiang 310056 (CN); LI, Yaowu, Hangzhou, Zhejiang 310056 (CN); WANG, Feng, Hangzhou, Zhejiang 310056 (CN)
(74) Representative: Elzaburu S.L.P.
(86) International application number: PCT/CN2023/140880
(87) International publication number: WO 2024/140449

(57) **Abstract**

An oxycycloalkyl-substituted camptothecin derivative used as an antibody-drug conjugate represented by formula (I) in the drug toxicity portion. The antibody-drug conjugate has a significant antitumor effect.

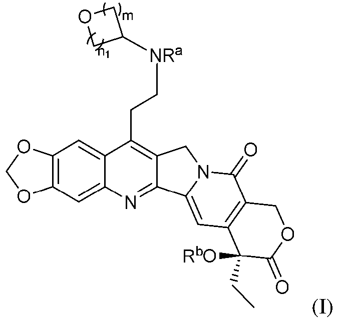

## Description

### TECHNICAL FIELD

The present disclosure relates to the medical field. Specifically, the present disclosure provides a series of antibody-drug conjugates from N-oxycycloalkyl substituted camptothecin derivatives, preparation method thereof, and use thereof in the anti-tumor field.

### BACKGROUND

Camptothecin (CPT) is a pentacyclic alkaloid isolated from the bark and trunk of *Camptotheca acuminata*/*Camptotheca*/Happy tree, which is native to China. Camptothecin inhibits topoisomerase I, which causes cell death. Due to the cytotoxic mechanism and broad-spectrum antitumor activity of camptothecin , considerable efforts have been made to develop clinical analogs of camptothecin.

Currently, researchers have done extensive work on using monoclonal antibodies to target and deliver small-molecule drugs to tumor cells. Although many different drug classes have been evaluated for antibody-mediated delivery, only a few have been proven to exhibit sufficient activity as antibody-drug conjugates while possessing appropriate toxicity profiles to warrant clinical development. One class of interest is camptothecin.

Antibody-drug conjugates (ADCs) are a class of potent agents effective in various abnormal cell growth or proliferative diseases (such as cancer). Antibody-drug conjugates typically consist of three distinct components: an antibody, a linker, and a cytotoxic component (small-molecule drug). The design of antibody-drug conjugates (ADC) is through the attachment of small-molecule drugs to antibodies typically via linkers, wherein the design of small-molecule drugs and their combinatorial adaptation with linkers and antigens are of vital importance. Currently, no existing technologies have investigated ADC drugs based on oxacyclyl-modified camptothecin and derivatives thereof. Exploration of drug technologies in this field holds significant value for the development of ADCs based on camptothecin and its derivative.

### SUMMARY

The purpose of the present disclosure is to provide an oxycycloalkyl-substituted camptothecin derivative used as an antibody-drug conjugate in the drug toxicity portion. These antibody-drug conjugate have significant antitumor effects.

One aspect of the present disclosure provides an antibody-drug conjugate represented by Formula (I), 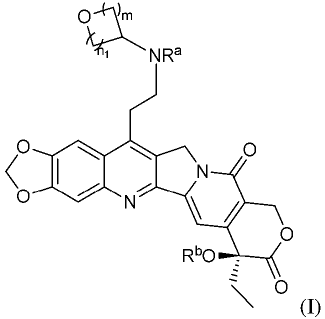 in the Formula,
m, n₁ are each independently selected from 1 or 2;
R^{a} is selected from hydrogen and -L¹-NH-L^{p}-Z-Ab, R^{b} is selected from hydrogen, -L²-NH-L^{p}-Z-Ab and -L³-NH-Z-Ab, provided that R^{a}, R^{b} are not both hydrogen;
wherein,
L¹ is selected from -C(=O)-L¹¹-L¹²-L¹³-;
L¹¹, L¹², L¹³ are each independently selected from -O-, C₁-C₃ alkylene and phenyl;
L² is selected from -C(=O)-NH-L²¹-CHR¹-;
L²¹ is selected from -C(=O)- and -NH-C(=O)-;
R¹ is selected from hydrogen, deuterium, C₁-C₆ alkyl and phenyl-substituted C₁-C₆ alkyl;
L³ is selected from -C(=O)-NR²-NR³-L³¹-L³²-;
R², R³ are each independently selected from hydrogen, deuterium and C₁-C₆ alkyl;
L³¹ is selected from -C(=O), -(C₁-C₃ alkylene)-C(=O), -5-8 membered alkyl-C(=O)-, -5-8 membered azaheteroaryl-C(=O)- and -O-(C₁-C₃ alkylene)-C(=O)-;
L³² is selected from -NH-(C₁-C₃ alkylene), -N(CH₃)₂-(C₁-C₃ alkylene) and -NH-(C₁-C₃ alkylene)-NH-;
L^{P} is a peptide residue consisting of 2 to 7 amino acids;
Z is selected from -L^{z}-L^{j}-, wherein, L^{z} is selected from -C(=O)-C₁-C₈ alkylene, -C(=O)-CH₂O-(CH₂CH₂O)₂₋₅-CH₂CH₂NH-, -C(=O)-(CH₂CH₂O)₂₋₆-CH₂CH₂- and -C(=O)-(CH₂CH₂O)₂₋₆-CH₂CH₂NH-, L^{j} is a linker that can conjugate to an antibody;
Ab is an antibody.

In the antibody-drug conjugate represented by Formula (I), the drug antibody ratio (DAR) thereof is selected from 2-8, further can be 3.5- 8, 6-8, or 6.2-7.6, for example, 3.95, 4.05, 6.2, 6.3, 6.4, 6.5, 6.6, 6.7, 6.8, 7.0, 7.1, 7.2, 7.3, 7.4, 7.5, or 7.6.

One aspect of the present disclosure further provides the antibody-drug conjugate represented by Formula (IA), (IB) or (IC) below, 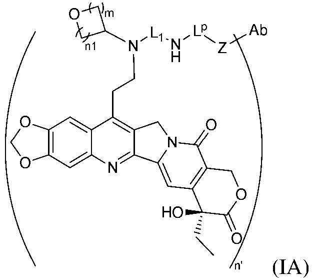 each group in the Formula is the same as defined above. 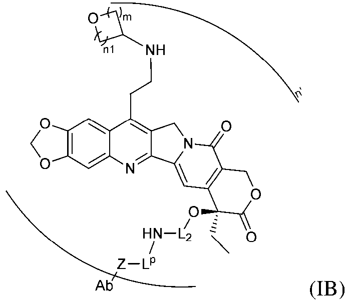 each group in the Formula is the same as defined above. 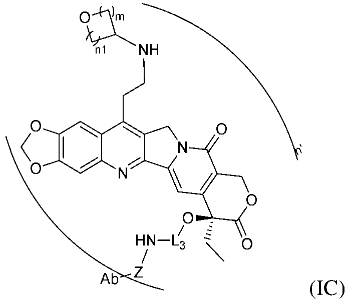 n' is selected from 2-8, further can be 3.5-8 or 6-8 or 6.2-7.6, for example can be 3.95, 4.05, 6.2, 6.3, 6.4, 6.5, 6.6, 6.7, 6.8, 7.0, 7.1, 7.2, 7.3, 7.4, 7.5 or 7.6.

Each group in the Formula is the same as defined above.

In some specific embodiments, the structure represented by 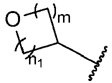 is selected from 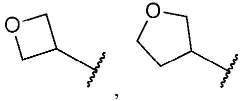 and

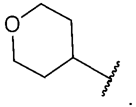

In some specific embodiments, L¹¹, L¹², L¹³ are each independently selected from -O-, methylene and phenyl.

In some specific embodiments, L¹¹-L¹²-L¹³ is selected from -O-CH₂-phenyl- and -CH₂-O-CH₂-.

In some specific embodiments, L¹ is selected from -C(=O)-O-CH₂-phenyl.

In some specific embodiments, R¹ is selected from hydrogen, deuterium, C₁-C₆ alkyl and phenyl-substituted C₁-C₆ alkyl.

In some specific embodiments, R¹ is selected from hydrogen, deuterium, methyl, isopropyl, isobutyl and phenylmethyl.

In some specific embodiments, R¹ is hydrogen.

In some specific embodiments, L² is selected from -C(=O)-NH-C(=O)-CH₂- and -CH₂-NH-NH-C(=O)-CH₂-.

In some specific embodiments, L² is -CH₂-NH-NH-C(=O)-CH₂-.

In some specific embodiments, R², R³ are each independently selected from hydrogen, deuterium and C₁-C₃ alkyl.

In some specific embodiments, R², R³ are each independently selected from hydrogen, deuterium and methyl.

In some specific embodiments, R² is hydrogen, R³ is hydrogen.

In some specific embodiments, L³¹ is selected from -C(=O), -methylene-C(=O), -phenyl-C(=O)-, -pyridyl-C(=O)- and - O-CH₂-C(=O)-.

In some specific embodiments, L³² is selected from -NH-CH₂-CH₂-, -N(CH₃)₂-CH₂-CH₂- and -NH-CH₂-NH-.

In some specific embodiments, L³ is -C(=O)-NH-NH-phenyl-C(=O)-NH-CH₂-CH₂-.

In some specific embodiments, the amino acid is selected from phenylalanine (Phe), glycine (Gly), valine (Val), alanine (Ala), leucine (Leu), lysine (Lys), citrulline (Cit), and asparagine (Asn).

In some specific embodiments, L^{p} is selected from peptide residues consisting of 2-5 amino acids selected from phenylalanine (Phe), glycine (Gly), valine (Val), alanine (Ala), leucine (Leu), lysine (Lys), citrulline (Cit), and asparagine (Asn).

In some specific embodiments, L^{p} is selected from peptide residues consisting of 2 or 3 amino acids selected from glycine (Gly), valine (Val), alanine (Ala), lysine (Lys), and citrulline (Cit).

In some specific embodiments, L^{p} is selected from Val-Cit, Val-Ala, Gly-Val-Ala, Gly-Val-Ala-Gly, Gly-Lys, Gly-Gly-Lys, Gly-Gly-Lys-Gly, Val-Ala, Ala-Ala-Asn, Gly-Leu, Gly-Gly-Leu, Gly-Gly-Leu-Gly, Gly-Phe, Gly-Gly-Phe and Gly-Gly-Phe-Gly.

In some specific embodiments, L^{p} is selected from 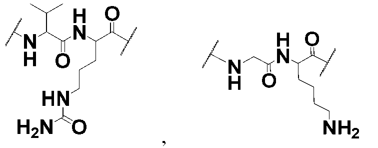 and 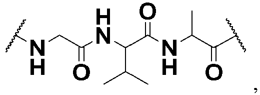 wherein the carbonyl terminus is linked to -NH- and the other terminus is linked to Z.

In some specific embodiments, L^{p} is selected from 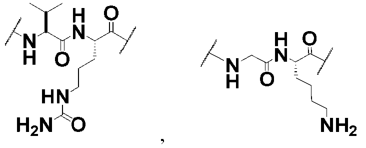 and 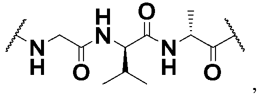 wherein the carbonyl terminus is linked to -NH- and the other terminus is linked to Z.

In some specific embodiments, L^{j} is selected from 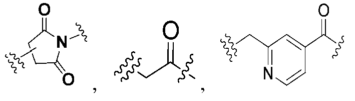 and 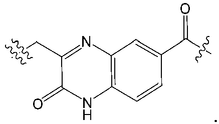 the position represented by " 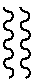 " is linked to the antibody, and the position represented by " 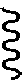 " is linked to the L^{Z} group.

In some specific embodiments, L^{z} is selected from -C(=O)-C₁-C₈ alkylene, -C(=O)-CH₂O-(CH₂CH₂O)₂₋₅-CH₂CH₂NH-, -C(=O)-(CH₂CH₂O)₂₋₆-CH₂CH₂- and -C(=O)-(CH₂CH₂O)₂₋₆-CH₂CH₂NH-.

In some specific embodiments, L^{z} is selected from -C(=O)-C₁-C₆ alkylene, -C(=O)-CH₂O-(CH₂CH₂O)₂₋₃-CH₂CH₂NH-, -C(=O)-(CH₂CH₂O)₂₋₄-CH₂CH₂- and -C(=O)-(CH₂CH₂O)₂₋₄-CH₂CH₂NH-.

In some specific embodiments, L^{z} is selected from -C(=O)-(CH₂)₅-, -C(=O)-CH₂O-(CH₂CH₂O)₃-CH₂CH₂NH-, -C(=O)-(CH₂CH₂O)₂-CH₂CH₂-, -C(=O)-(CH₂CH₂O)₂-CH₂CH₂NH- and -C(=O)-(CH₂CH₂O)₄-CH₂CH₂NH-.

In some specific embodiments, Z is selected from 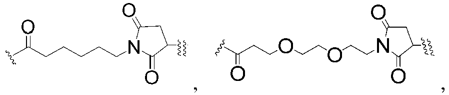 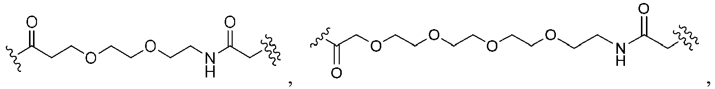 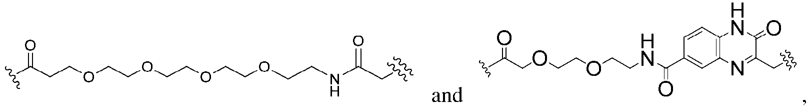 the position represented by " 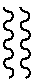 " is linked to the antibody, and the position represented by " 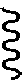 " is linked to the L^{p} group or L³ group.

In some specific embodiments, the antibody is an antibody against tumor-associated antigen.

In some specific embodiments, the antibody against tumor-associated antigen is selected from anti-Her2 antibody, anti-Trop2 antibody, anti-B7H3 antibody, anti-5T4 antibody, anti-Nectin-4 antibody, anti-CD20 antibody, and anti-ROR1 antibody.

In some specific embodiments, the antibody-drug conjugate represented by Formula (I) has a structure represented by Formula (I-1) or (I-1'), 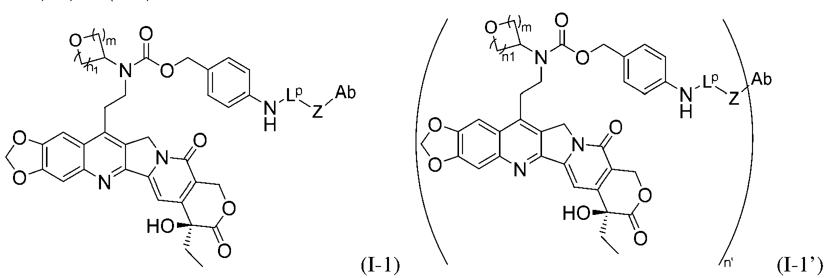 in the Formula, m, n₁, L^{p}, Z, Ab are each defined the same as the compound of Formula (I), n' is defined the same as above.

In some specific embodiments, the antibody-drug conjugate represented by Formula (I) has a structure represented by Formula (I-2) or Formula (I-2), Formula (I-3) or Formula (I-3'), 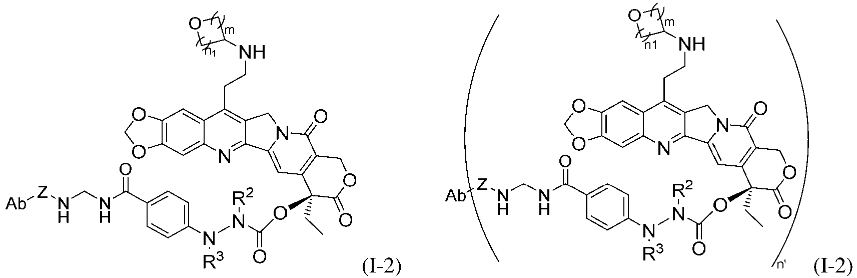 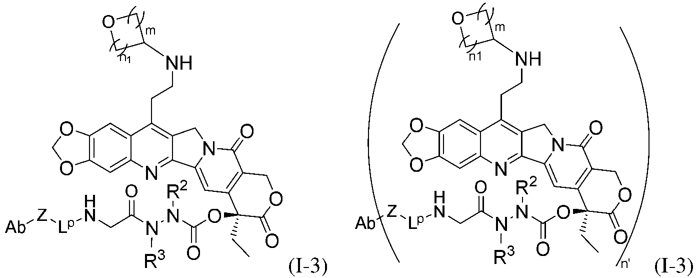 in the Formula, R², R³, m, n₁, L^{p}, Z, Ab are each defined the same as the compound of Formula (I), n' is defined the same as above.

The present disclosure provides the following antibody-drug conjugate: 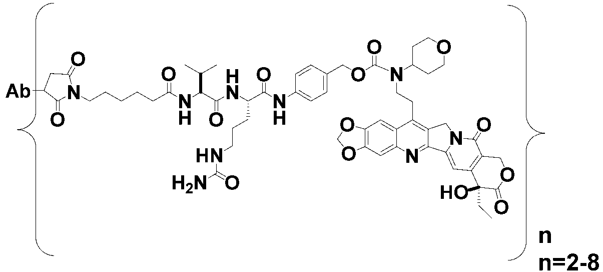 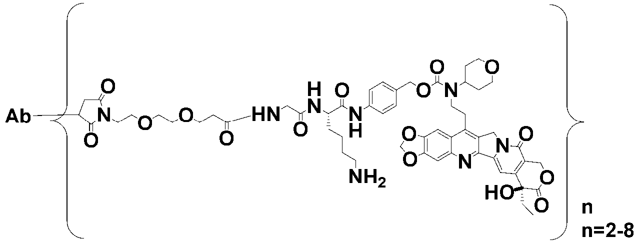 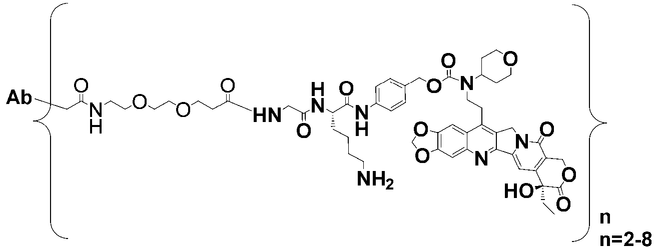 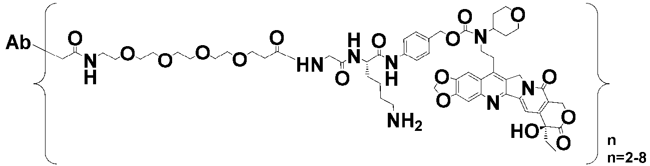 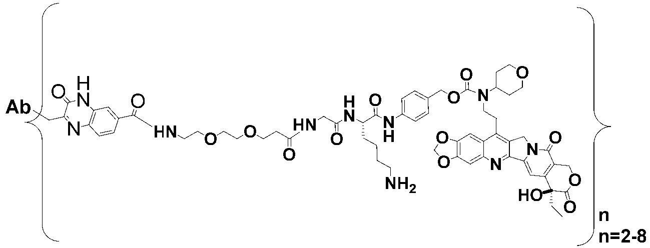 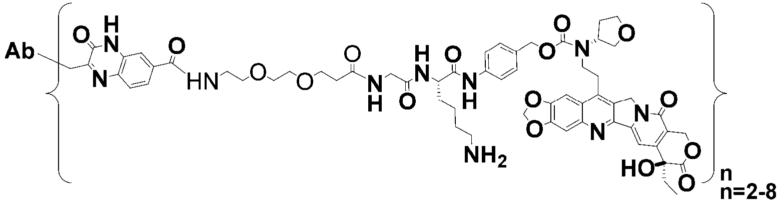 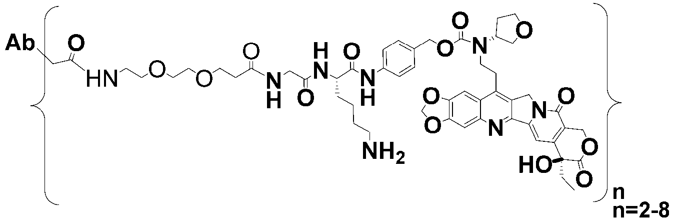 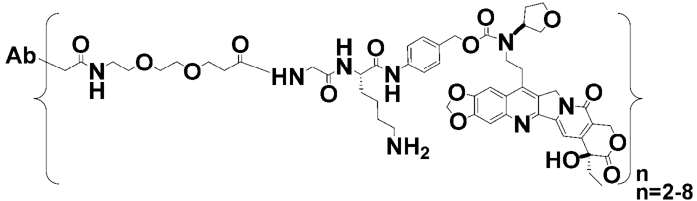 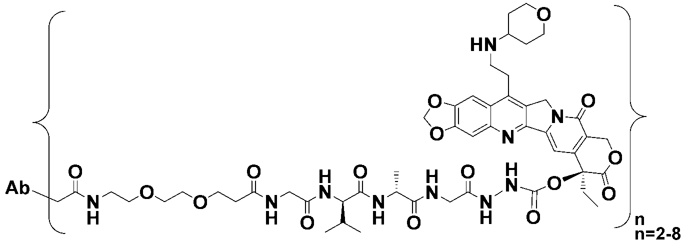 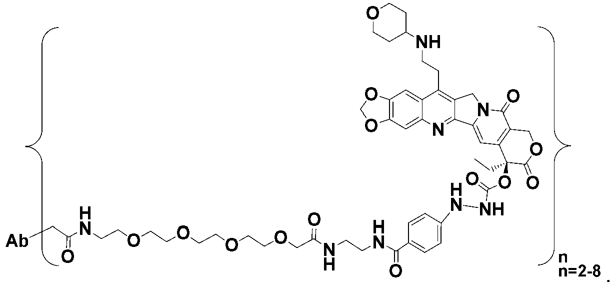 wherein, Ab is defined the same as the compound of Formula (I).

In some specific embodiments, Ab is selected from antibodies against tumor-associated antigens, and further selected from anti-Her2 antibodies, anti-Trop2 antibodies, anti-B7H3 antibodies, anti-5T4 antibodies, anti-Nectin-4 antibodies, anti-CD20 antibodies and anti-ROR1 antibodies.

In some specific embodiments, Ab is the HS627 antibodies or the IP140B antibodies, wherein the heavy chain amino acid sequence of HS627 is as set forth in SEQ ID NO: 1, and the light chain amino acid sequence thereof is as set forth in SEQ ID NO: 2; the heavy chain amino acid sequence of the IP140B antibodies is as set forth in SEQ ID NO: 3, and the light chain amino acid sequence thereof is as set forth in SEQ ID NO: 4.

In one embodiment, n is selected from 2-8, -further can be 3.5-8, or 6-8, or 6.2-7.6. For example can be 3.95, 4.05, 6.2, 6.3, 6.4, 6.5, 6.6, 6.7, 6.8, 7.0, 7.1, 7.2, 7.3, 7.4, 7.5 or 7.6.

The present disclosure provides the following antibody-drug conjugate: 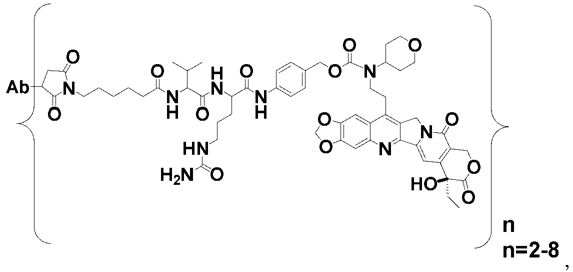 further, n is selected from 6.2-6.4. 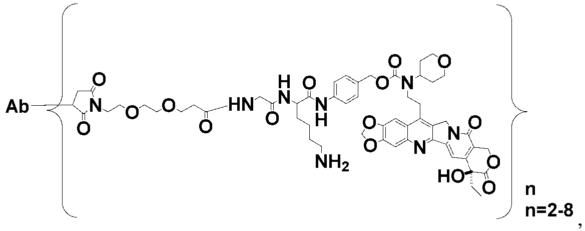 further, n is selected from 6.7-6.8. 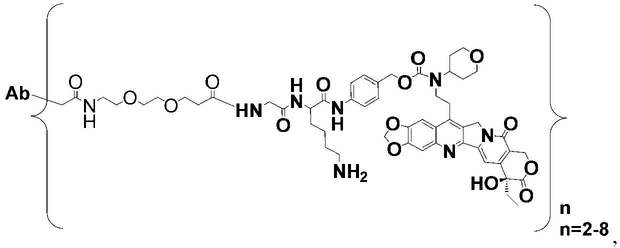 further, n is selected from 3.95-7.2, for example, can be 3.95-4.05 or n=7.1-7.2. 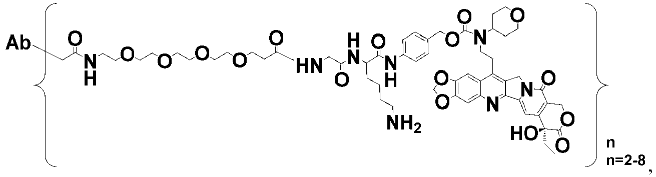 further, n is selected from 7.0-7.1. 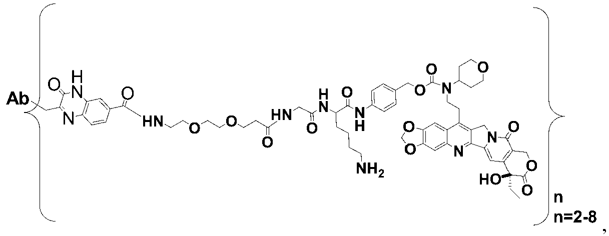 further, n is selected from 6.8-7.0. 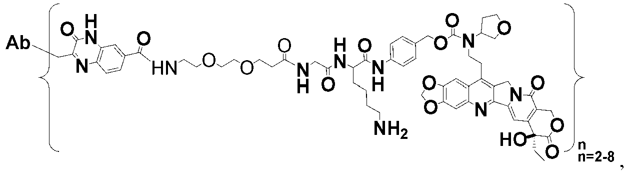 further, n is selected from 7.4-7.5. 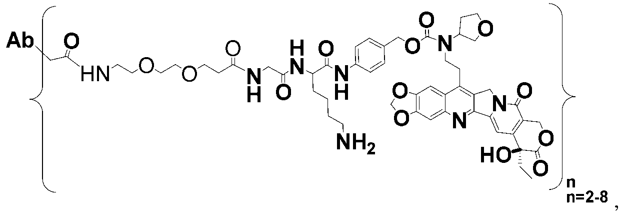 further, n is selected from 7.4-7.5. 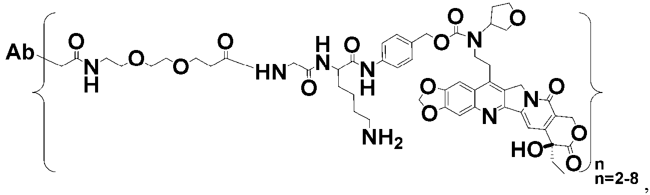 further, n is selected from 7.4-7.5. 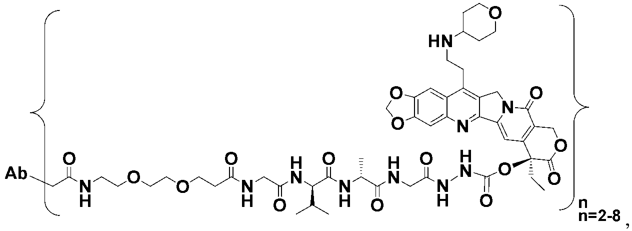 further, n is selected from 7.3-7.5. 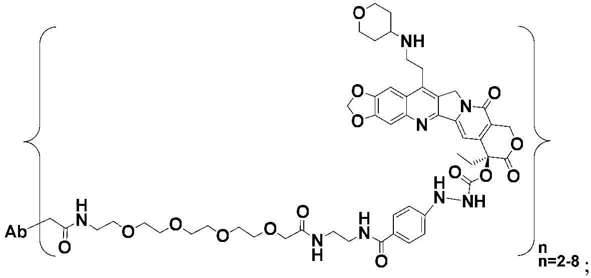 further, n is selected from 7.4-7.5.
wherein, Ab is defined the same as the compound of Formula (I).

Another aspect of the present disclosure provides a method for preparing the above antibody-drug conjugate, which is selected from the following synthetic routes:
synthetic route 1:
   a compound of Formula (a) and a compound of Formula (b) were reacted to afford a compound of Formula (c), and the compound of Formula (c) was conjugated with an antibody to afford the compound of Formula (I); wherein, R^{a} is -L¹-NH-L^{p}-Z-Ab, R^{b} is hydrogen; 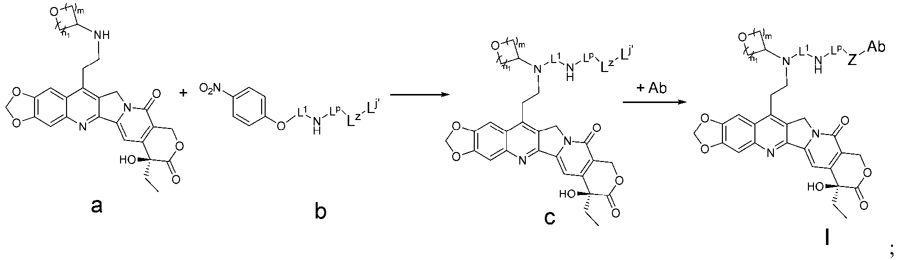
synthetic route 2:
   a compound of Formula (d) was condensed with HO-L^{z}-L^{j}' to afford a compound of Formula (e), and the compound of Formula (e) was conjugated with an antibody to afford the compound of Formula (I); wherein, R^{a} is -L¹-NH-L^{p}-Z-Ab, R^{b} is hydrogen; 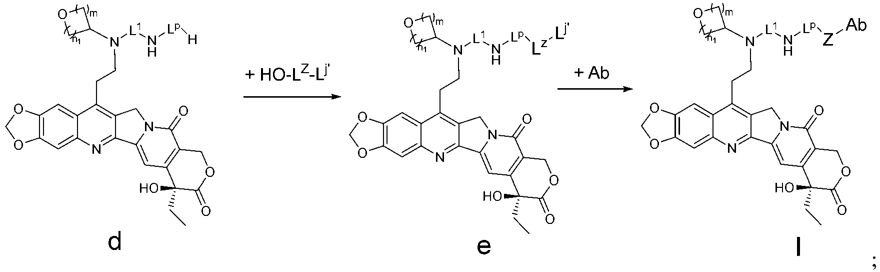
synthetic route 3:
   a compound of Formula (f) was condensed with HO-L^{j}' to afford a compound of Formula (g), and the compound of Formula (g) was conjugated with an antibody to afford the compound of Formula (I); wherein, R^{a} is -L¹-NH-L^{p}-Z-Ab, R^{b} is hydrogen; 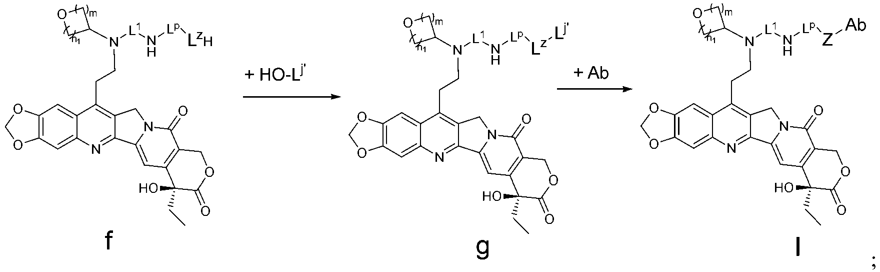
synthetic route 4:
   a compound of Formula (h) was condensed with HO-L^{z}-L^{j}' to afford a compound of Formula (i), after removing an amino protecting group X from the compound of Formula (i), the resulting compound was conjugated with an antibody to afford the compound of Formula (I); wherein, R^{a} is hydrogen, R^{b} is selected from -L²-NH-L^{p}-Z-Ab; 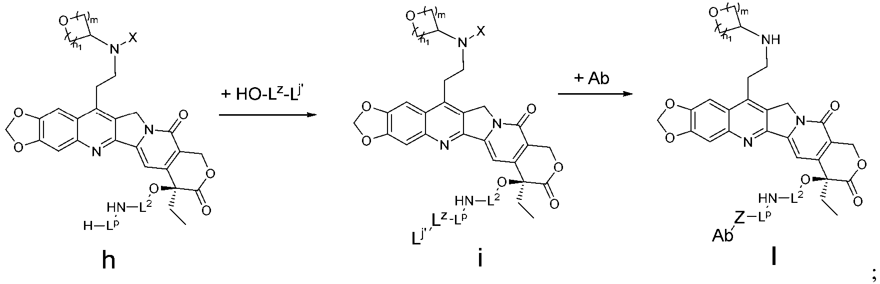
synthetic route 5:
   a compound of Formula (j) was condensed with HO-L^{z}-L^{j}' to afford a compound of Formula (k), after removing an amino protecting group X from the compound of Formula (k), the resulting compound was conjugated with an antibody to afford the compound of Formula (I); wherein, R^{a} is hydrogen, R^{b} is selected from -L³-NH-Z-Ab; 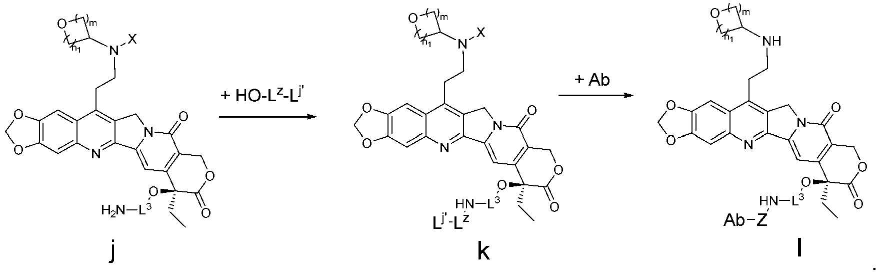
in the above synthetic routes, m, n₁, L¹, L², L³, L^{p}, L^{z}, Ab are each defined the same as the compound of Formula (I);
L^{j}' is selected from 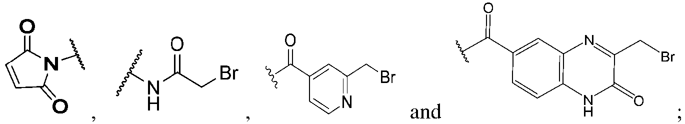
X is an amino protecting group.

Another aspect of the present disclosure provides a pharmaceutical composition comprising the above antibody-drug conjugate or the antibody-drug conjugate prepared by the above method and a pharmaceutically acceptable carrier.

Another aspect of the present disclosure provides a use of the above antibody-drug conjugate, the antibody-drug conjugate prepared by the above method, or the above pharmaceutical composition in the manufacture of an anti-tumor medicament.

Another aspect of the present disclosure provides a method for treating or curing a tumor disease in a patient in need thereof, comprising administering to the patient the above antibody-drug conjugate or pharmaceutical composition.

In some specific embodiments, the dosage of the above antibody-drug conjugate or pharmaceutical composition is a therapeutically effective amount.

In some specific embodiments, the tumor is selected from a solid tumor.

In some specific embodiments, the tumor is selected from esophageal cancer, lung cancer or breast cancer.

In some specific embodiments, the lung cancer is non-small cell lung cancer.

### Description of Drawings

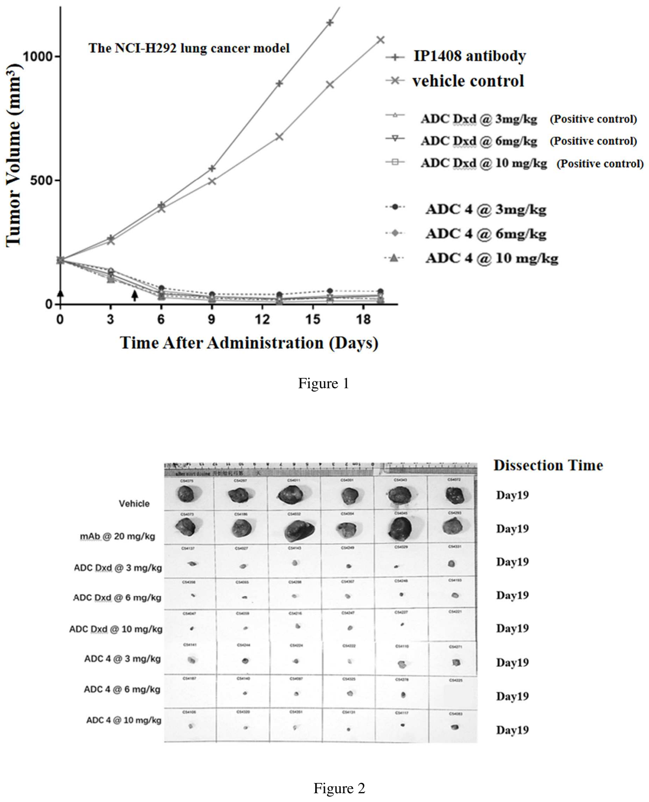

Figure 1 is a graph of the *in vivo* tumor growth curve for ADC4 in inhibiting the NCI-H292 human lung cancer CDX model in Assay 2.
Figure 2 is a photograph of tumors after dissection from the NCI-H292 human lung cancer CDX model inhibited by ADC4 in Assay 2.

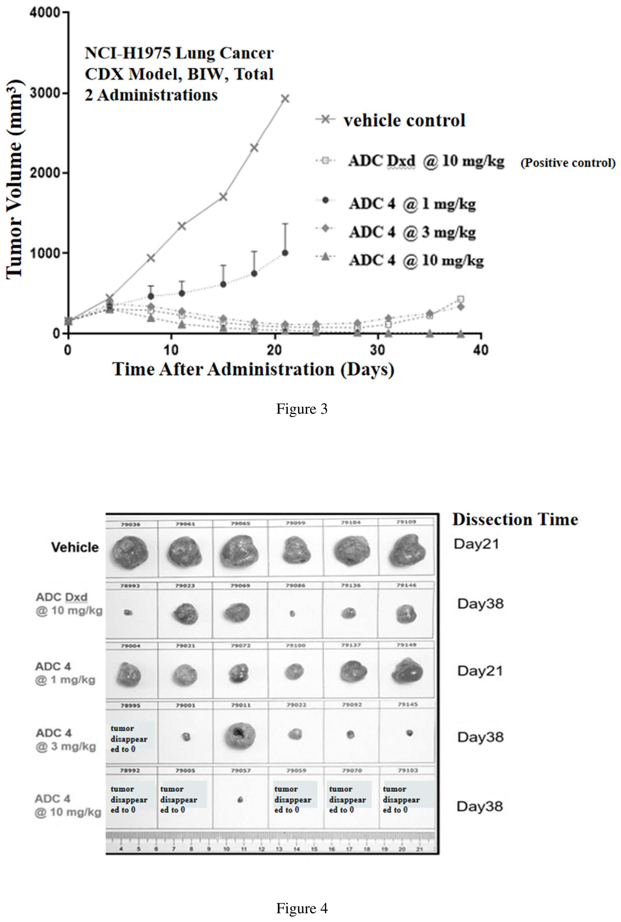

Figure 3 is a graph of the *in vivo* tumor growth curve for ADC4 in inhibiting the NCI-H1975 human lung cancer CDX model in Assay 2.
Figure 4 is a photograph of tumors after dissection from the NCI-H1975 human lung cancer CDX model inhibited by ADC4 in Assay 2.

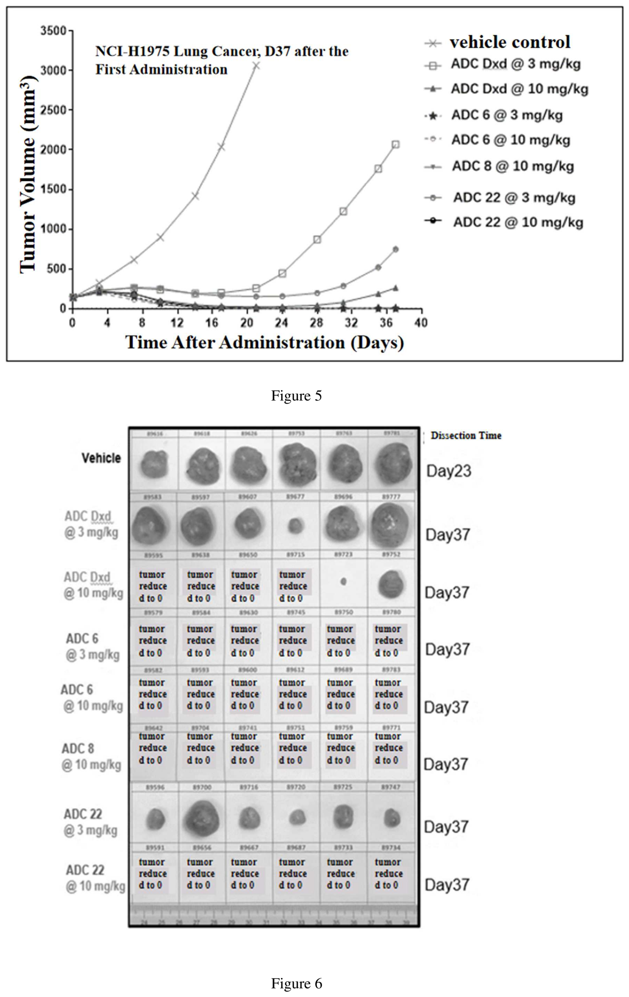

Figure 5 is a graph of the *in vivo* tumor growth curves for ADC6, 8, and 22 in inhibiting the NCI-H1975 human lung cancer CDX model in Assay 2.
Figure 6 is a photograph of tumors after dissection from the NCI-H1975 human lung cancer CDX model inhibited by ADC6, 8, and 22 in Assay 2.

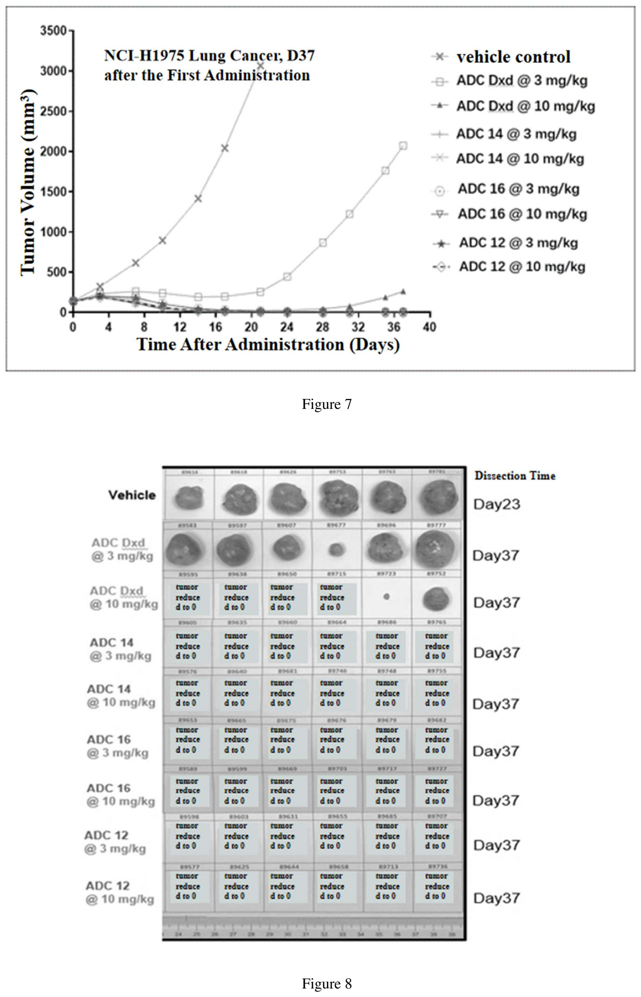

Figure 7 is a graph of the *in vivo* tumor growth curves for ADC14, 16, and 12 in inhibiting the NCI-H1975 human lung cancer CDX model in Assay 2.
Figure 8 is a photograph of tumors after dissection from the NCI-H1975 human lung cancer CDX model inhibited by ADC14, 16, and 12 in Assay 2.

### DETAILED DESCRIPTION OF THE EMBODIMENTS

### I. Definition

In the present disclosure, unless otherwise specified, scientific and technical terms used herein have the meanings commonly understood by those skilled in the art. Also, the relevant terms and laboratory procedures used herein are terms and conventional procedures widely used in the corresponding fields. Meanwhile, for a better understanding of the present disclosure, definitions and explanations of related terms are provided below.

As used herein and unless otherwise specified, the terms "comprising", "including", "having", "containing", including grammatical equivalents thereof, should generally be understood to be open-ended and non-limiting, for example, not excluding other unrecited elements or steps.

The compound of the present disclosure can be asymmetric, for example, having one or more stereoisomers. Unless otherwise specified, all stereoisomers are included, for example, enantiomers and diastereomers. The stereoisomers include geometric isomers (such as cis and trans structures) and optical isomers (such as enantiomers), therapeutic substances consisting of monomers, racemates, racemic mixtures and pharmaceutically acceptable salts thereof. The compound of the present disclosure containing asymmetric carbon atoms can be isolated in optically active pure form or racemic form. Optically active pure forms can be resolved from racemic mixtures or synthesized by using chiral starting materials or chiral reagents. Racemates, diastereomers, enantiomers are all included within the scope of the present disclosure.

A numerical range as used herein refers to each integer within the given range. For example,"C1-C6" refers to the group that can have 1 carbon atom, 2 carbon atoms, 3 carbon atoms, 4 carbon atoms, 5 carbon atoms or 6 carbon atoms.

When any variable (such as Rn) appears more than once in the composition or structure of a compound, the definition thereof is independent in each case. Therefore, for example, if a group is substituted with 1-5 R's, then the group can optionally be substituted with up to 5 R's, and each R has independent options in every case. In addition, combinations of substituents and/or variants thereof are only allowed if such combinations result in the formation of stable compounds.

The term "C₁₋C₆ alkyl" refers to straight or branched alkyl groups having 1 to 6 carbon atoms. Specific examples of alkyl groups include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, and various branched isomers thereof, etc.

" 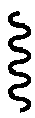 " refers to the chemical bond junction. It should be noted that the structural fragments described in the disclosure (such as -L11-L12-L12- or -Lz-Lj-), unless otherwise specified, represent the sequential linking of corresponding groups from left to right. For example, when -L¹²- is C₁-C₂ alkylene-O-, it means the left side of C₁-C₂ alkylene-O- is linked to -L¹¹-, and the right end is linked to -L¹³-.

Understandably, in the antibody-drug conjugates of the present disclosure, such as in formulas (I), (I-1), (I-2), and (I-3), the "-" between the drug-linker fragment and the antibody is intended to signify the linking relationship between the antibody and the fragment, and is not intended to limit the conjugate to one antibody linked to one drug-linker fragment. As is known in the art, due to the presence of multiple interchain disulfide bonds on an antibody, one antibody can be linked to one or more drugs.

### Medicament or pharmaceutical composition

The drugs or drug compositions of the present disclosure can be administered orally, topically, parenterally, or mucosally (e.g., sublingually, by inhalation, or rectally) in dose unit formulations comprising conventional non-toxic pharmaceutically acceptable carriers. An oral route is generally preferred. The active agents can be administered orally in the form of capsules, tablets, etc.

The term "treatment" includes inhibiting, alleviating, preventing, or eliminating one or more symptoms or side effects associated with the disease, condition, or disorder being treated.

The term "inhibition" is used in relation to controls. Those skilled in the art will readily determine the appropriate control for each experiment. For example, a reduced response in subjects or cells treated with the compound is compared to the response in subjects or cells not treated with the compound.

The term "patient" refers to an animal, preferably a mammal, and more preferably a human.

The term "pharmaceutical composition" refers to a composition that includes the compound or pharmaceutically acceptable salt thereof of the present disclosure, as well as at least one pharmaceutically acceptable component selected from the following, depending on the administration route and the nature of dosage form, including but not limited to: carriers, diluents, adjuvants, excipients, preservatives, fillers, disintegrants, wetting agents, emulsifiers, suspension agents, sweeteners, flavoring agents, fragrances, antimicrobials, antifungals, lubricants, dispersing agents, temperature-sensitive materials, temperature regulators, adhesives, stabilizers, and suspending agents.

The term "effective amount" or "therapeutically effective amount" refers to a sufficient amount of a drug or medication that is non-toxic but can achieve the desired effect. In embodiments of the present disclosure, when treating a patient in accordance with the present disclosure, the amount of drugs administered depends on many factors, such as the specific dosing regimen, the type of disease or condition and the severity thereof, the uniqueness (for example, body weight) of the treated person or host to be treated, but the amount of drug administered depends on specific surrounding circumstances, including, for example, the specific drug that has been employed, the route of administration, the treated condition, and the treated person or host to be treated, the administered dose may be routinely determined by methods known in the art. Generally, regarding the dosage used for adult therapy, the administered dose is typically within the range of 0.02-5000mg/day, for example, the range of about 1-1500mg/day. The required dose can be conveniently administered as a single dose, or as doses given simultaneously (or in a short period of time) or in divided doses at appropriate intervals, such as two, three, four doses per day or more. It can be understood by those skilled in the art that, although the above dose ranges are provided, the specific effective amount can be appropriately adjusted based on the patient's condition and in conjunction with a physician's diagnosis.

The term "antibody-drug conjugate (ADC)" refers to a biologically active small molecule drug linked to a monoclonal antibody via a chemical linker. The monoclonal antibody serves as a carrier to target and deliver the small molecule drug to specific target cells. As used herein, the term "antibody-drug conjugate" has the same meaning as antibody-drug conjugate.

The term "Drug Antibody Ratio (DAR)" refers to the average number of payload molecules (antitumor compounds or drugs) attached to a single monoclonal antibody.

### Abbreviations:

### Fmoc: 9-fluorenylmethoxycarbonyl.

Amino acids constituting the peptide residue L^{P}: Val: valine, whose structural formula is 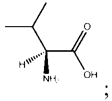 Ala: alanine, whose structural formula is 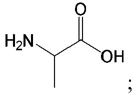 Gly: glycine, whose structural formula is 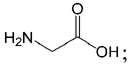 Phe: phenylalanine, whose structural formula is

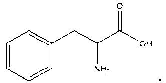

L^{P} can be obtained by amino acid condensation methods known in the art.

PABC: 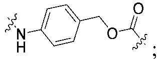 ha: 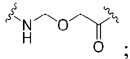 m:

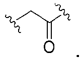

In the present disclosure, the antibody and the linker L^{j} for connecting to the antibody can be linked by methods known in the art. For example, when the structure of L^{j} is 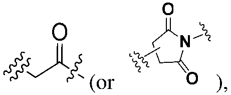 it can be linked to a reactive group such as a thiol on the antibody through 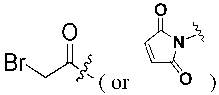 to afford, which is not particularly limited in the present disclosure.

### II. Example

In the example, the preparation of ADC uses, but not limited to, pertuzumab and IP140B antibodies. The heavy chain amino acid sequence of pertuzumab (HS627, PERTUZUMAB) is as follows (SEQ ID NO: 1):

EVQLVESGGGLVQPGGSLRLSCAASGFTFTDYTMDWVRQAPGKGLEWVADVNPNSGGSIYNQRFKGRFTLS
VDRSKNTLYLQMNSLRAEDTAVYYCARNLGPSFYFDYWGQGTLVTVSSASTKGPSVFPLAPSSKSTSGGTAALGCL
VKDYFPEPVTVSWNSGALTSGVHTFPAVLQSSGLYSLSSVVTVPSSSLGTQTYICNVNHKPSNTKVDKKVEPKSCD
KTHTCPPCPAPELLGGPSVFLFPPKPKDTLMISRTPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHEALHNHYTQKSLSLSPG

The light chain amino acid sequence is as follows (SEQ ID NO: 2):

DIQMTQSPSSLSASVGDRVTITCKASQDVSIGVAWYQQKPGKAPKLLIYSASYRYTGVPSRFSGSGSGTDFTLTI
SSLQPEDFATYYCQQYYIYPYTFGQGTKVEIKRTVAAPSVFIFPPSDEQLKSGTASVVCLLNNFYPREAKVQWKVDN
ALQSGNSQESVTEQDSKDSTYSLSSTLTLSKADYEKHKVYACEVTHQGLSSPVTKSFNRGEC

The heavy chain amino acid sequence of the IP140B antibody is as follows (SEQ ID NO: 3):

QVQLVQSGGGLVQPGGSLRLSCAASGFTFSSYEMNWVRQAPGKGLEWVSYISSSGSTIYYADSVKGRFTISRD

NAKNSLYLQMNSLRAEDTAVYYCAREMQFGWELLGAFDIWGQGTMVTVSSASTKGPSVFPLAPSSKSTSGGTAAL
GCLVKDYFPEPVTVSWNSGALTSGVHTFPAVLQSSGLYSLSSVVTVPSSSLGTQTYICNVNHKPSNTKVDKRVEPKS
CDKTHTCPPCPAPELLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSRDELTKNQVSLTCLVKG
FYPSDIAVEWESNGQPENNYKTTPPVLDSDGSFFLYSKLTVDKSRWQQGNVFSCSVMHEALHNHYTQKSLSLSPGK

The light chain amino acid sequence is as follows (SEQ ID NO: 4):

EIVLTQSPGTLSLSPGERATLSCRASQSVSSSYLAWYQQKPGQAPRLLIYGASSRATGIPDRFSGSGSGTDFTLTI
SRLEPEDFAVYYCQQYGSSFGQGTKLEIKRTVAAPSVFIFPPSDEQLKSGTASVVCLLNNFYPREAKVQWKVDNAL
QSGNSQESVTEQDSKDSTYSLSSTLTLSKADYEKHKVYACEVTHQGLSSPVTKSFNRGEC.

Unless otherwise specified, the raw materials and equipment used in the specific embodiments of the present disclosure are all known products, obtained by purchasing commercially available products.

### Intermediate HX-1a: 7-(2-(Tetrahydropyran-4-yl)amino)ethyl-10,11-methylenedioxycamptothecin

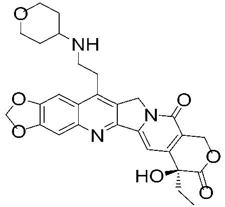

4-Aminotetrahydropyran (0.75 g, 7.4 mmol), concentrated hydrochloric acid (0.7 mL, 7.5 mmol), and DMSO (15 mL) were reacted and the mixture was warmed to 120°C in an oil bath. Then 7-methyl-10,11-methylenedioxycamptothecin (0.5 g, 1.2 mmol) was added, and the mixture was warmed to 130-140°C, then reacted for 1 h. Methanol was added, and the mixture was filtered. The filtrate was concentrated and purified by silica gel column chromatography to afford the title compound as a gray solid.(0.41 g, yield 63%, HPLC 96%); ¹H NMR (500 MHz, DMSO-d₆) δ 7.59 (s, 1H), 7.47-7.41 (m, 1H), 7.25-7.14 (m, 1H), 6.54 (s, 1H), 6.27 (t, *J*=9.9 Hz, 2H), 5.77 (s, 1H), 5.44 (d, *J*=16.5 Hz, 2H), 5.23-5.11 (m, 2H), 3.83 (d, *J*=9.5 Hz, 2H), 3.23 (t, *J*=24.9 Hz, 4H), 2.93 (d, *J*=32.7 Hz, 3H), 1.99-1.72 (m, 4H), 1.33 (s, 2H), 0.96-0.81 (m, 3H); ¹³C NMR(126 MHz, DMSO) δ 173.04, 157.26, 151.30, 150.58, 149.68, 149.42, 147.49, 146.73,146.68, 128.52, 124.79, 118.49, 105.90, 103.14, 99.83, 96.39, 72.82, 66.05, 65.69, 55.41, 53.65, 50.31, 30.66, 8.27; LC-MS (M+H) + 520.11 (theoretical value 519.20).

### Intermediate HX-6a: 7-(2-(N-(S)-tetrahydrofuran-3-yl)amino)ethyl-10,11-methylenedioxycamptothecin

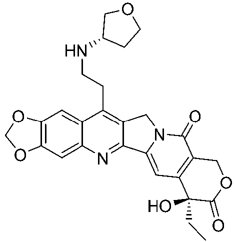

(S)-3-Aminotetrahydrofuran (64.31 mg, 0.738 mmol), concentrated hydrochloric acid (0.05 mL, 0.6 mmol), DMSO (1.5 mL), and 7-methyl-10,11-methylenedioxycamptothecin (50 mg, 0.12 mmol) were added to a reaction flask. The mixture was stirred and heated to 120-130 °C and reacted for 1 h, then cooled to room temperature. Methyl tert-butyl ether was added, and the precipitated solid was filtered and purified by silica gel column chromatography to afford the title compound.(18 mg, yield 33%, HPLC 95.9%); 1H NMR (500 MHz, DMSO-d6) δ 7.61 (d, J=3.4 Hz, 1H), 7.47 (d, J=3.9 Hz, 1H), 7.22 (d, J=4.2 Hz,1H), 6.50 (s, 1H), 6.29 (d, J=4.0 Hz, 2H), 5.42 (d, J=4.0 Hz, 2H), 5.21 (d, J=3.2 Hz, 2H), 3.87-3.58 (m, 4H), 3.21 (s, 2H), 2.85 (td, J=11.2, 10.7, 5.8 Hz, 2H), 1.90 (ddt, J=29.9, 10.7, 6.2 Hz, 3H), 1.68-1.60 (m, 1H), 1.08-0.76 (m, 3H); 13C NMR (126 MHz, DMSO) δ 173.0, 157.3, 151.3, 150.6, 149.7, 149.4, 147. 6, 146.9, 141.2, 128.6, 124.9, 118.5, 105.9, 103.1, 100.0, 96.4, 72.9, 72.7, 66.9, 65.7, 58.4, 50.4, 47.6, 32.6, 30.7, 30.6, 8.2; LC-MS (M+H)+ 506.31 (theoretical value 505.18).

### Intermediate HX-8a: 7-(2-(N-(R)-tetrahydrofuran-3-yl)amino)ethyl-10,11-methylenedioxycamptothecin

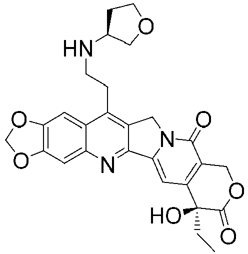

(R)-3-Aminotetrahydrofuran (64.31 mg, 0.738 mmol), concentrated hydrochloric acid (0.05 mL, 0.6 mmol), DMSO (1.5 mL), and 7-methyl-10,11-methylenedioxycamptothecin (50 mg, 0.12 mmol) were added to a reaction flask. The mixture was stirred and heated to 120-130 °C and reacted for 1 h, then cooled to room temperature. Methyl tert-butyl ether was added, and the precipitated solid was filtered and purified by silica gel column chromatography to afford the title compound(16.2 mg, yield 32.3%, HPLC 96.2%); 1H NMR (500 MHz, DMSO-d6) δ 7.61 (s, 1H), 7.47 (s, 1H), 7.22 (s, 1H), 6.50 (s, 1H), 6.28 (s, 2H), 5.42 (s, 2H), 5.21 (s, 2H), 4.21-3.53 (m, 4H), 3.21 (t, J=7.6 Hz, 2H), 2.84 (q, J=8.7, 8.1Hz, 2H), 1.90 (ddt, J=31.9, 16.2, 7.1 Hz, 3H), 1.63 (dd, J=12.3, 6.1 Hz, 1H), 0.88 (t, J=7.3 Hz, 3H); 13C NMR (126 MHz, DMSO) δ 173.0, 157.3, 151.3, 150.6, 149.7, 149.4, 147. 5, 146.8, 141.2, 128.6, 124.9, 118.4, 105.9, 103.1, 99.9, 96.4, 72.9, 66.9, 65.7, 58.4, 55.4, 50.4, 47.6, 32.6, 30.7, 30.6, 8.3; LC-MS (M+H)+ 506.34 (theoretical value 505.18).

### Example 1 : 7-(N-(HS627-mc-Val-Cit-PABC), N-(tetrahydropyran-4-yl)amino)ethyl-10,11-methylenedioxycamptothecin (1)

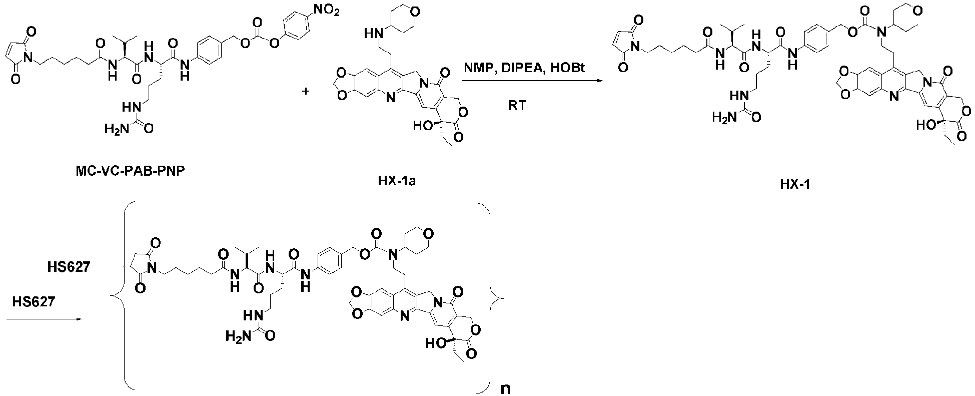

NMP (0.2 mL) was added to a reaction flask, then compound mc-VC-PAB-PNP (20 mg, 0.027 mmol), DIPEA (17.52 mg, 0.135 mmol), **HX-1a** (14.08 mg, 0.027 mmol), and HOBt (7.33 mg, 0.054 mmol) were added in sequence. The mixture was reacted at room temperature for 1 h. The reaction solution was injected into a 25 g C18 pre-column (pre-equilibrated with acetonitrile, then with water, the water phase contains 0.1% TFA), then eluted via medium-pressure reverse-phase C18 chromatography (gradient: 10% to 55% acetonitrile in water over 50 min). The product was lyophilized to afford HX-1 as a yellow solid (20.2 mg, yield 66%). LCMS: (M+1)+ 1118.31 (theoretical value: 1117.48).

HS627 antibody (20.0 mg/mL, 10 mg, 0.066 mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-1** (0.89 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 1** (3 mg/mL, 2 mL).

UV-HPLC calculated average value: n=6.20.

### Example 2: 7-(N-(IP140B-mc-Val-Cit-PABC), N-(tetrahydropyran-4-yl)amino)ethyl-10,11-methylenedioxycamptothecin (2)

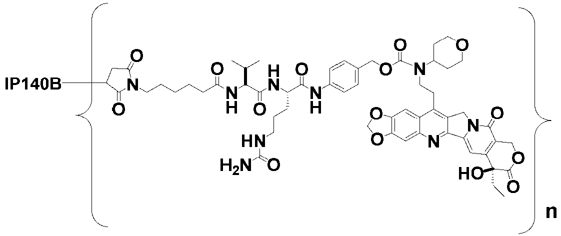

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-1** (0.89mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 2** (3.2mg/mL, 2mL).

UV-HPLC calculated average value: n=6.40.

### Example 3: 7-(N-(HS627-mPEG₂-Gly-Lys-PABC), N-(tetrahydropyran-4-yl)amino)ethyl-10,11-methylenedioxycamptothecin (3)

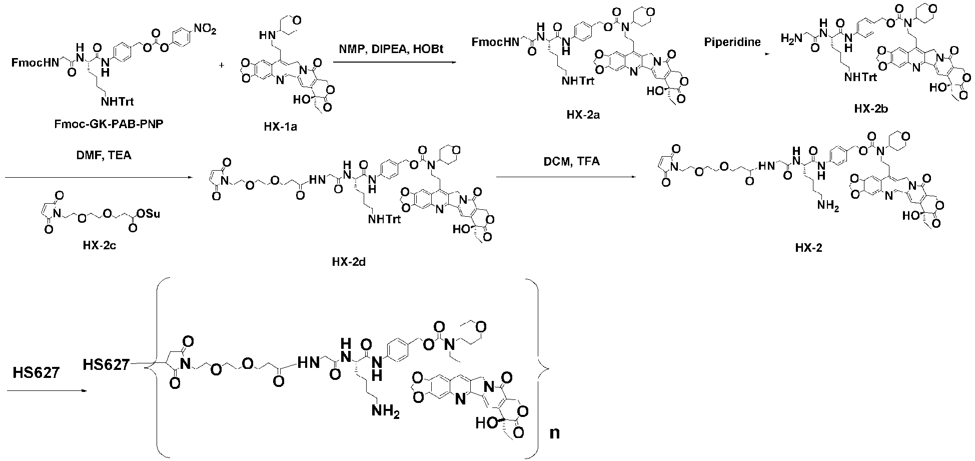

In a 10 mL single-neck flask, **HX-1a** (40 mg, 0.077 mmol), 0.4 mL of NMP, DIPEA (50 mg, 0.38 mmol), Fmoc-GK-PAB-PNP (73 mg, 0.077 mmol), and HOBt (10.4 mg, 0.077 mmol) were added in sequence. The mixture was stirred at room temperature for 0.5 h to afford **HX-2a.** Then, 0.4 mL of piperidine (V:V=10%) was added, and the mixture was reacted at room temperature for 0.5 h. 30 mL of Methyl tert-butyl ether was added to the reaction mixture, which was centrifuged, and the supernatant was removed. The solvent was removed under reduced pressure to afford the solid product **HX-2b** (69 mg, yield 77%), which was used directly in the next step. LCMS: (M+1)⁺ 1096.45 (theoretical value: 1095.47).

In a 10 mL single-neck flask, DCM (2 mL), **HX-2b** (69 mg, 0.063 mmol), and **HX-2c** (27 mg, 0.075 mmol) were added in sequence. The mixture was stirred for 2 min, then TEA (6.40 mg, 0.063 mmol) was added, and the mixture was continued stirring to react at room temperature for 15 min. 3g of silica gel was added to the reaction solution for column chromatography purification to afford intermediate **HX-2d.** (72 mg, yield 85%), LCMS: (M+1)⁺ 1335.48 (theoretical value: 1334.55).

In a 10 mL single-neck flask, DCM (1 mL), **HX-2d** (72 mg, 0.053 mmol), and TFA (0.1 mL) were added in sequence. The mixture was stirred to react at room temperature for 20 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 15% to 40% acetonitrile in water over 30 min). The product was lyophilized to afford **HX-2** as a yellow solid (29 mg, yield 50%); LCMS: (M+1)⁺ 1093.16 (theoretical value: 1092.44).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-2** (0.87mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 3** (2.9 mg/mL, 2 mL).

UV-HPLC calculated average value: n=6.80.

### Example 4: 7-(N-(IP140B-mPEG₂-Gly-Lys-PABC), N-(tetrahydropyran-4-yl)amino)ethyl-10,11-methylenedioxycamptothecin (4)

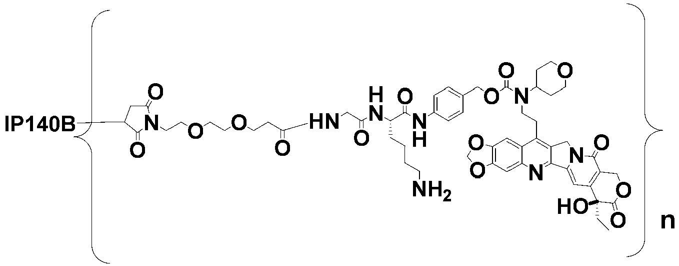

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-2** (0.87 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 4** (3mg/mL, 2mL).

UV-HPLC calculated average value: n=6.70.

### Example 5: 7-(N-(HS627-AcPEG₂-Gly-Lys-PABC), N-(tetrahydropyran-4-yl)amino)ethyl-10,11-methylenedioxycamptothecin (5)

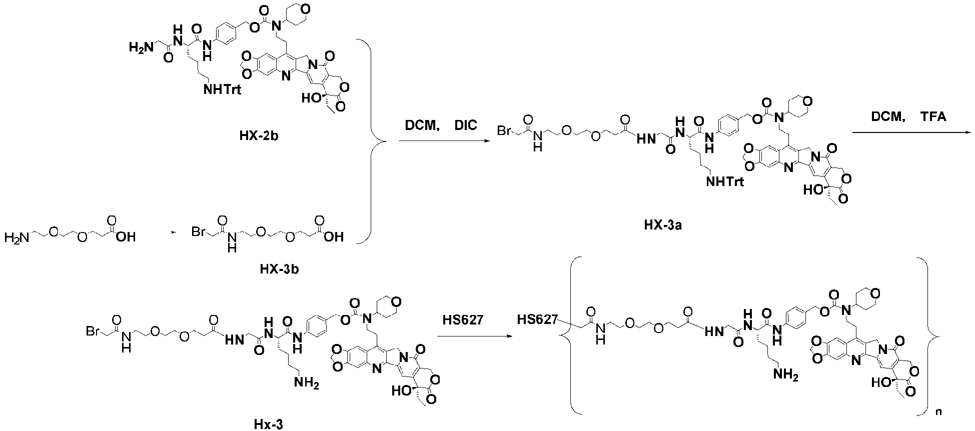

In a 50 mL single-neck flask, DCM (20 mL), 3-(2-(2-aminoethoxy)ethoxy)propionic acid (2 g, 11.3 mmol), and bromoacetyl bromide (2.27 g, 11.3 mmol) were added in sequence. The mixture was stirred for 30 min, then 10 mL of saturated sodium chloride was added to the reaction solution for extraction. The DCM phase was dried over anhydrous magnesium sulfate to afford intermediate **HX-3b.** (3.1g, yield 89%), LCMS: (M+1)⁺ 298.00 (theoretical value: 297.02).

In a 10 mL single-neck flask, DCM (1mL), **HX-2b** (50mg, 0.045mmol), **HX-3b** (13.6 mg, 0.045 mmol) and DIC (5.75 mg, 0.045 mmol) were added in sequence. The mixture was stirred to react at room temperature for 60 min, then 0.1 mL of TFA was added to the reaction solution, and the mixture was continued to react for 30 min. Finally, the reaction solution was injected into a 25 g C18 pre-column (pre-equilibrated with acetonitrile, then with water, the water phase contains 0.1% TFA) , then eluted via medium-pressure reverse-phase C18 chromatography (gradient: 15% to 40% acetonitrile in water over 30min). The product was lyophilized to afford **HX-3** as a white solid (16 mg, yield 31%); LCMS: (M+1)⁺ 1133.21 (theoretical value: 1132.38).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-3** (0.91 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 5** (3 mg/mL, 2 mL).

UV-HPLC calculated average value: n=7.2.

### Example 6: 7-(N-(IP140B-AcPEG₂-Gly-Lys-PABC), N-(tetrahydropyran-4-yl)amino)ethyl-10,11-methylenedioxycamptothecin (6)

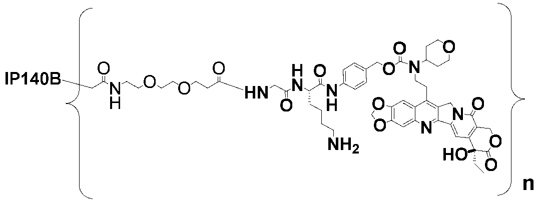

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-3** (0.91mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 6** (3.1 mg/mL, 2 mL).

UV-HPLC calculated average value: n=7.10.

### Example 7: 7-(N-(HS627-AcPEG₄-Gly-Lys-PABC), N-(tetrahydropyran-4-yl)amino)ethyl-10,11-methylenedioxycamptothecin (7)

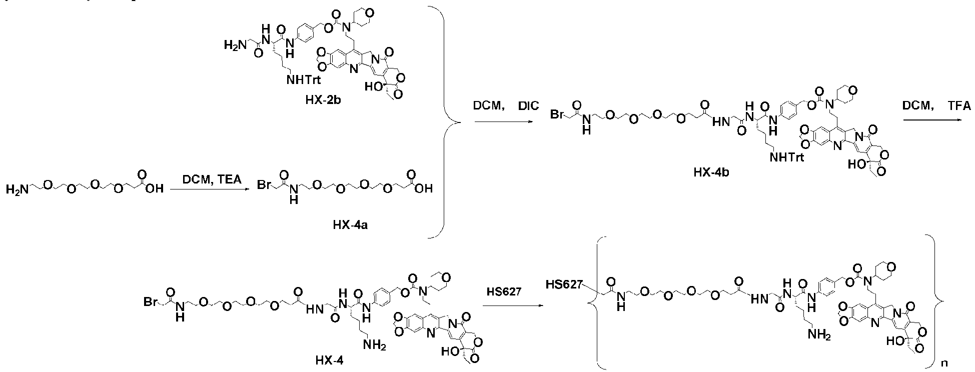

In a 50 mL single-neck flask, DCM (30 mL), 1-amino-3,6,9,12-tetraoxapentadecan-15-oic acid (2 g, 7.5 mmol), and bromoacetyl bromide (1.51 g, 7.5 mmol) were added in sequence. The mixture was stirred for 30 min, then 15 mL of saturated sodium chloride was added for extraction. The DCM phase was dried over anhydrous magnesium sulfate to afford intermediate **HX-4a.** (2.5 g, yield 85%), LCMS: (M+1)⁺ 386.24 (theoretical value: 385.07).

In a 10 mL single-neck flask, DCM (1mL), **HX-2b** (50 mg, 0.045 mmol), **HX-4a** (17.4 mg, 0.045 mmol) and DIC (5.75 mg, 0.045 mmol) were added in sequence. The mixture was stirred to react at room temperature for 60 min, then 0.1 mL of TFA was added to the reaction solution, and the mixture was continued to react for 30 min. Finally, the reaction solution was injected into a 25 g C18 pre-column (pre-equilibrated with acetonitrile, then with water, the water phase contains 0.1% TFA) , then eluted via medium-pressure reverse-phase C18 chromatography (gradient: 15% to 40% acetonitrile in water over 30min) . The product was lyophilized to afford **HX-4** as a white solid (16 mg, yield 31%); LCMS: (M+1)⁺ 1223.05 (theoretical value: 1222.15).

HS627 antibody (20.0mg/mL, 10 mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-4** (0.98 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 7** (3.2 mg/mL, 2 mL).

UV-HPLC calculated average value: n=7.1.

### Example 8: 7-(N-(IP140B-AcPEG4-Gly-Lys-PABC), N-(tetrahydropyran-4-yl)amino)ethyl-10,11-methylenedioxycamptothecin (8)

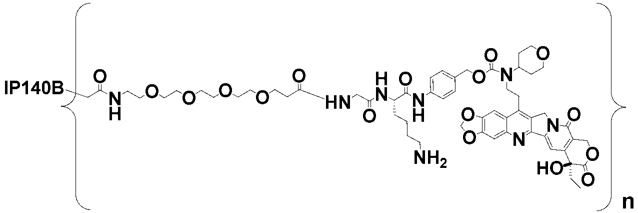

IP140B antibody (20.0mg/mL, 10 mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-4** (0.98 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford the antibody-drug conjugate **ADC 8** (3.2 mg/mL, 2 mL).

UV-HPLC calculated average value: n=7.0.

### Example 9: 7-(N-(HS627-Qx-PEG2-Gly-Lys-PABC), N-(tetrahydropyran-4-yl)amino)ethyl-10,11-methylenedioxycamptothecin (9)

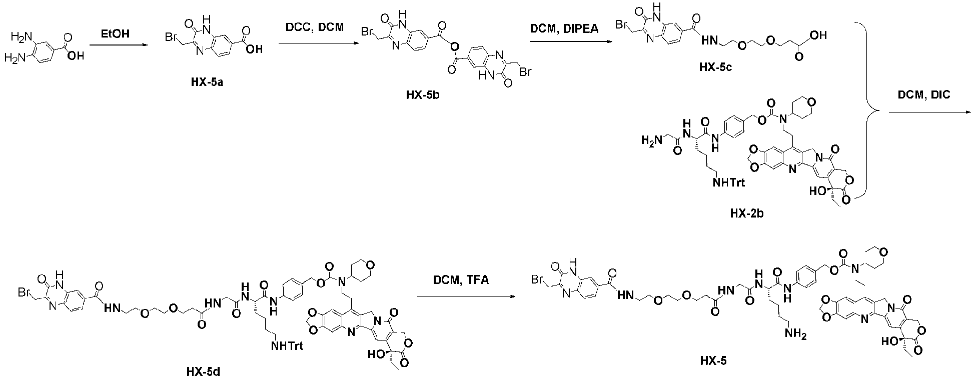

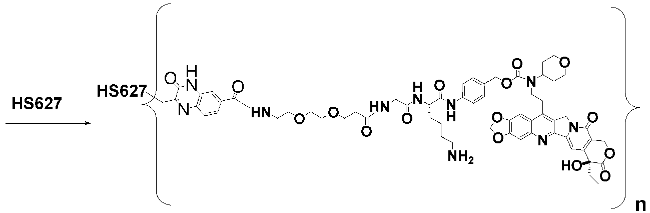

In a 250 mL single-neck flask, 3,4-diaminobenzoic acid (5 g, 0.032 mol) was dispersed in 65 mL of ethanol. Methyl bromopyruvate (5.95 g, 0.032 mol) was added, and the mixture was stirred at room temperature for 1 h. The solid was filtered, dried, and purified by silica gel column chromatography to afford the product **HX-5a** as a yellow solid. (8 g, yield 83%); LCMS: (M+1)⁺ 283.09 (theoretical value: 281.96).

In a 50 mL single-neck flask, DCM (10 mL), **HX-5a** (1 g, 3.53 mmol), and DCC (0.36 g, 1.76 mmol) were added in sequence. The mixture was stirred to react at room temperature for 60 min, then filtered to remove DCU, to afford intermediate **HX-5b** (0.96 g, yield 100%), which was used directly in the next step. LCMS: (M+2)⁺ 548.15 (theoretical value: 545.92).

In a 50 mL single-neck flask, DCM (10mL), **HX-5b** (0.96 g, 1.76 mmol), 3-(2-(2-aminoethoxy)ethoxy)propionic acid (0.31g, 1.76mmol) and TEA (0.17 g, 1.76 mmol) were added in sequence. The mixture was stirred to react at room temperature for 60 min. The reaction solution was injected into a 40 g C18 pre-column (pre-equilibrated with acetonitrile, then with water, the water phase contains 0.1% TFA) , then eluted via medium-pressure reverse-phase C18 chromatography (gradient: 0% to 40% acetonitrile in water over 30min). The product was lyophilized to afford **HX-5c** as a pink solid (1.21 g, yield 80%); LCMS: (M+1)⁺ 442.27 (theoretical value: 441.05).

In a 10 mL single-neck flask, DCM (1mL), **HX-2b** (50 mg, 0.045 mmol), **HX-5c** (19.89 mg, 0.045 mmol) and DIC (5.75 mg, 0.045 mmol) were added in sequence. After stirring to react at room temperature for 90 min, HPLC indicated complete consumption of the starting materials. Then, 0.1 mL of TFA was added to the reaction solution, and the mixture was continued to react for 30 min. Finally, the reaction solution was injected into a 25 g C18 pre-column (pre-equilibrated with acetonitrile, then with water, the water phase contains 0.1% TFA), then eluted via medium-pressure reverse-phase C18 chromatography (gradient: 15% to 40% acetonitrile in water over 30min). The product was lyophilized to afford **HX-5** as a white solid (16 mg, yield 31%); LCMS: (M+1)⁺ 1277.31 (theoretical value: 1276.46).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-5** (1 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 9** (3.3 mg/mL, 2 mL).

UV-HPLC calculated average value: n=6.8.

### Example 10: 7-(N-(IP140B-Qx-PEG2-Gly-Lys-PABC), N-(tetrahydropyran-4-yl)amino)ethyl-10,11-methylenedioxycamptothecin (10)

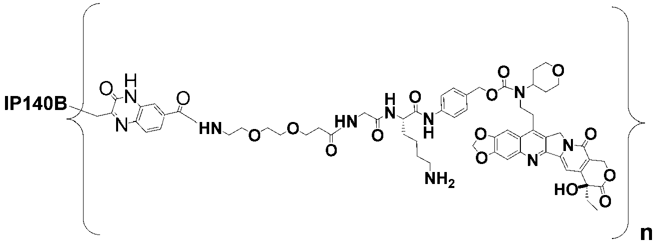

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-5** (1 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM histidine acetate buffer, 250mM sorbitol, 0.02% Tween 80, pH5.7, to afford the antibody-drug conjugate **ADC 10** (3.2 mg/mL, 2 mL).

UV-HPLC calculated average value: n=7.0.

### Example 11: 7-(N-(HS627-Qx-PEG2-Gly-Lys-PABC), N-((R)-tetrahydrofuran-3-yl)amino)ethyl-10,11-methylenedioxycamptothecin (11)

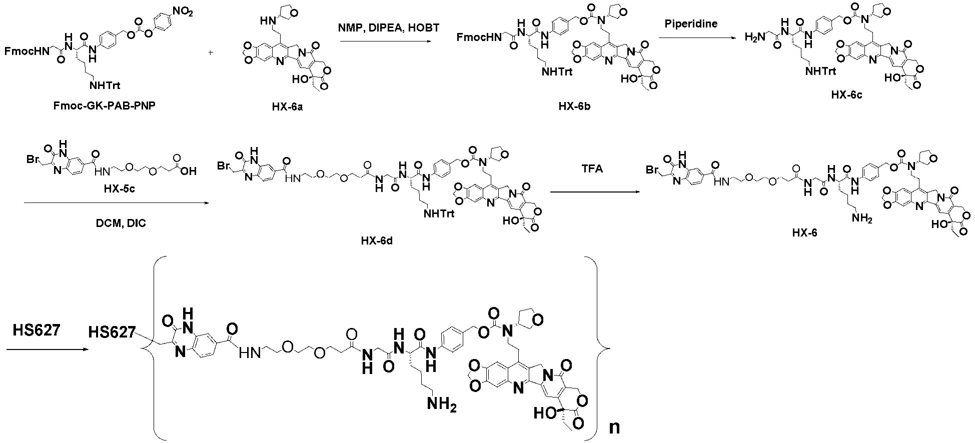

In a 10 mL single-neck flask, **HX-6a** (50 mg, 0.099 mmol), 1 mL of NMP, DIPEA (64 mg, 0.49 mmol), Fmoc-GK-PAB-PNP (93 mg, 0.099 mmol) and HOBt (13.3 mg, 0.099 mmol) were added in sequence. The mixture was stirred at room temperature for 0.5 h to afford **HX-6b,** which was used directly in the next step without purification; LCMS: (M+1)⁺ 1304.47 (theoretical value: 1303.53).

To the above reaction solution of **HX-6b,** 0.1 mL of piperidine (10% v/v) was added, and the mixture was reacted at room temperature for 0.5 h. Then, 20 mL of methyl tert-butyl ether was added to the reaction solution, which was centrifuged and the supernatant was removed. The solvent was removed under reduced pressure to afford the product **HX-6c** as a solid (61 mg, yield 56%), which was used directly in the next step. LCMS: (M+1)⁺ 1082.22 (theoretical value: 1081.46).

In a 10 mL single-neck flask, DCM (1 mL), **HX-6c** (61 mg, 0.056 mmol), **HX-5c** (24.7 mg, 0.056 mmol) and DIC (7.05 mg, 0.056 mmol) were added in sequence. The mixture was stirred to react at room temperature for 90 min, then 0.1 mL of TFA was added to the reaction solution, and the mixture was continued to react for 30 min. Finally, the reaction solution was injected into a 25 g C18 pre-column (pre-equilibrated with acetonitrile, then with water, the water phase contains 0.1% TFA) , then eluted via medium-pressure reverse-phase C18 chromatography (gradient: 5% to 40% acetonitrile in water over 30min) . The product was lyophilized to afford **HX-6** as a white solid (21 mg, yield 29%); LCMS: (M+1)⁺ 1264.15 (theoretical value: 1262.39).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-6** (1 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 11** (3.1 mg/mL, 2 mL).

UV-HPLC calculated average value: n=7.4.

### Example 12: 7-(N-(IP140B-Qx-PEG2-Gly-Lys-PABC), N-((R)-tetrahydrofuran-3-yl)amino)ethyl-10,11-methylenedioxycamptothecin (12)

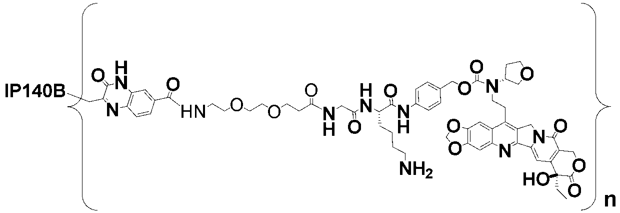

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-6** (1 mg, 0.8 mmol) was dissolved in 0.1 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC 12** (3.4 mg/mL, 2 mL).

UV-HPLC calculated average value: n=7.5.

### Example 13: 7-(N-(HS627-Ac-PEG2-Gly-Lys-PABC), N-((R)-tetrahydrofuran-3-yl)amino)ethyl-10,11-methylenedioxycamptothecin (13)

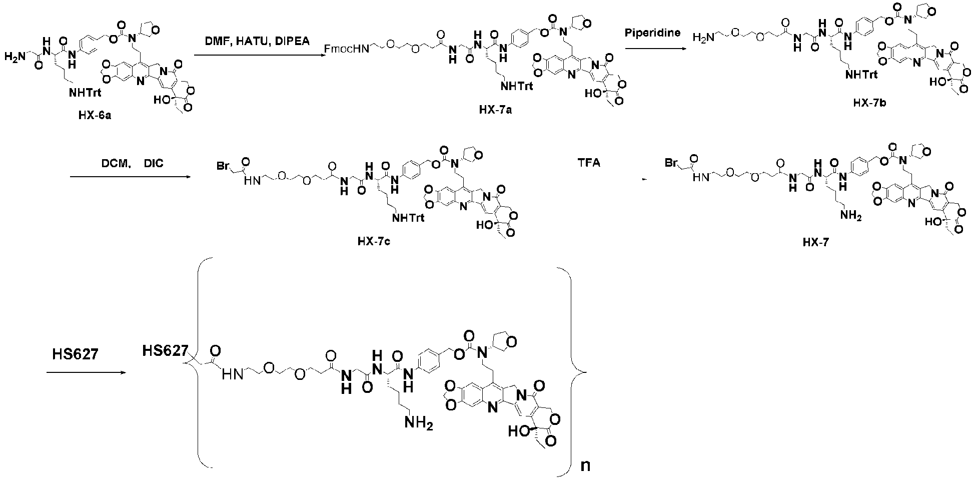

In a 10 mL single-neck flask, **HX-6a** (50 mg, 0.046 mmol), 1 mL of DMF, Fmoc-NH-PEG2-CH₂CH₂COOH (18 mg, 0.046 mmol) and HATU (17.57 mg, 0.046 mmol) were added in sequence. The mixture was stirred at room temperature for 1 h, then purified by silica gel column chromatography to afford the product **HX-7a** as a yellow powder. (55 mg, yield 82%); LCMS: (M+1)⁺ 1463.51 (theoretical value: 1462.62).

**HX-7a** (55 mg, 0.037 mmol) was dissolved in 0.2 mL of DMF reaction solution. Then, 0.2 mL of piperidine (V:V=10%) was added, and the mixture was reacted at room temperature for 0.5 h. 30 mL of Methyl tert-butyl ether was added to the reaction mixture, which was centrifuged, and the supernatant was removed. The solvent was removed under reduced pressure to afford the product **HX-7b as a solid** (35 mg, yield 76%) which was used directly in the next step. LCMS: (M+1)⁺ 1241.41 (theoretical value: 1240.55).

In a 10 mL single-neck flask, DCM (1mL), **HX-7b** (35 mg, 0.028 mmol), bromoacetic acid (24.7 mg, 0.056 mmol) and DIC (7.05 mg, 0.056 mmol) were added in sequence. The mixture was stirred to react at room temperature for 90 min, then 0.1 mL of TFA was added to the reaction solution, and the mixture was continued to react for 30 min. Finally, the reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30min). The product was lyophilized to afford **HX-7** as a yellow solid (16 mg, yield 57%); LCMS: (M+1)⁺ 1119.24 (theoretical value: 1118.36).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-7** (0.9 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 13** (3.1 mg/mL, 2 mL).

UV-HPLC calculated average value: n=7.5.

### Example 14: 7-(N-(IP140B-Ac-PEG2-Gly-Lys-PABC), N-((R)-tetrahydrofuran-3-yl)amino)ethyl-10,11-methylenedioxycamptothecin (14)

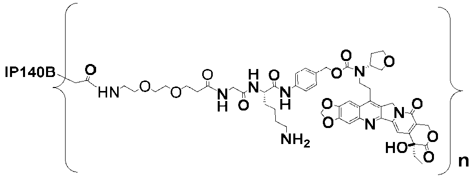

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-7** (0.9mg, 0.8mmol) was dissolved in 0.09 mL of DMA and added to the above solution system. After mixing well, the mixture was rotated to react at room temperature for 2 h.After the reaction was completed, the buffer was exchanged using a NAP-5 gel column (Cytiva) into 20 mM histidine solution, 250mM sorbitol, 0.02% Tween 80, pH5.7, to afford the antibody-drug conjugate **ADC 14** (3.0 mg/mL, 2 mL).

UV-HPLC calculated average value: n=7.4.

### Example 15: 7-(N-(HS627-Ac-PEG2-Gly-Lys-PABC), N-((S)-tetrahydrofuran-3-yl)amino)ethyl-10,11-methylenedioxycamptothecin (15)

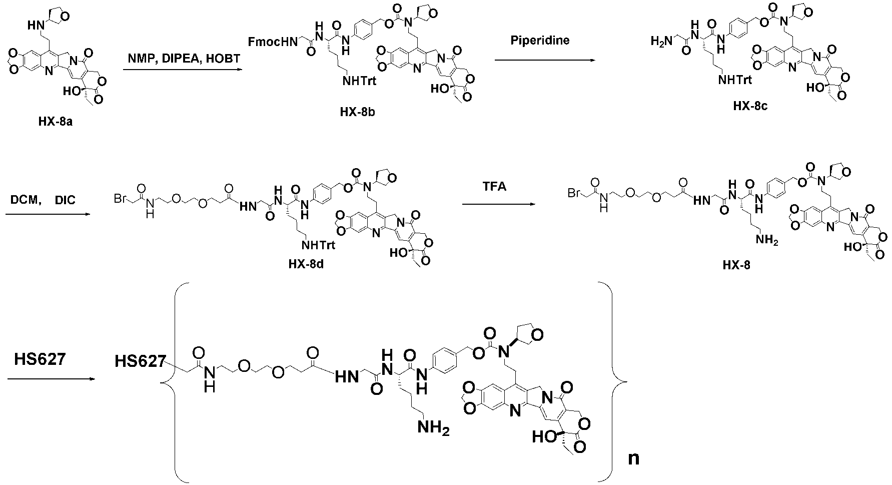

In a 10 mL single-neck flask, **HX-8a** (60 mg, 0.118 mmol), 1 mL of NMP, DIPEA (46 mg, 0.356 mmol), Fmoc-GK-PAB-PNP (80 mg, 0.118 mmol), and HOBt (16 mg, 0.118 mmol) were added in sequence. The mixture was stirred to react at room temperature for 4 h to afford the reaction solution of **HX-8b,** which was used directly in the next step. LCMS: (M+1)⁺ 1304.45 (theoretical value: 1303.53).

To the above reaction solution of **HX-8b,** 0.1 mL of piperidine (10% v/v) was added, and the mixture was reacted at room temperature for 0.5 h. Then, 20 mL of methyl tert-butyl ether was added to the reaction solution, which was centrifuged and the supernatant was removed. The solvent was removed under reduced pressure to afford **HX-8c** as a solid (61 mg, yield 56%), which was used directly in the next step. LCMS: (M+1)⁺ 1082.22 (theoretical value: 1081.46).

In a 10 mL single-neck flask, DCM (1mL), **HX-8c** (90 mg, 0.083 mmol), **HX-3b** (24 mg, 0.083 mmol) and DIC (10.45 mg, 0.083 mmol) were added in sequence. The mixture was stirred to react at room temperature for 90 min, then 0.1 mL of TFA was added to the reaction solution, and the mixture was continued to react for 30 min. Finally, the reaction solution was injected into a 25 g C18 pre-column (pre-equilibrated with acetonitrile, then with water, the water phase contains 0.1% TFA) , then eluted via medium-pressure reverse-phase C18 chromatography (gradient: 5% to 40% acetonitrile in water over 30min). The product was lyophilized to afford **HX-8** as a yellow solid (32 mg, yield 34%); LCMS: (M+1)⁺ 1119.23 (theoretical value: 1118.36).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-8** (0.9 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 15** (3.2 mg/mL, 2 mL).

UV-HPLC calculated average value: n=7.4.

### Example 16: 7-(N-(IP140B-Ac-PEG2-Gly-Lys-PABC), N-((S)-tetrahydrofuran-3-yl)amino)ethyl-10,11-methylenedioxycamptothecin (16)

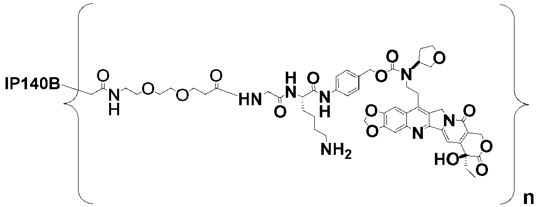

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-8** (0.9 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250mM sorbitol, 0.02% Tween 80, pH 5.7, to afford the antibody-drug conjugate **ADC 16** (3.3 mg/mL, 2 mL).

UV-HPLC calculated average value: n=7.5.

### Example 17: 20-O-(HS627-Ac-PEG2-Gly-Val-Ala-Gly-NHNHC)-7-(N-(tetrahydropyran-4-yl)amino)ethyl-10,11-methylenedioxycamptothecin (17)

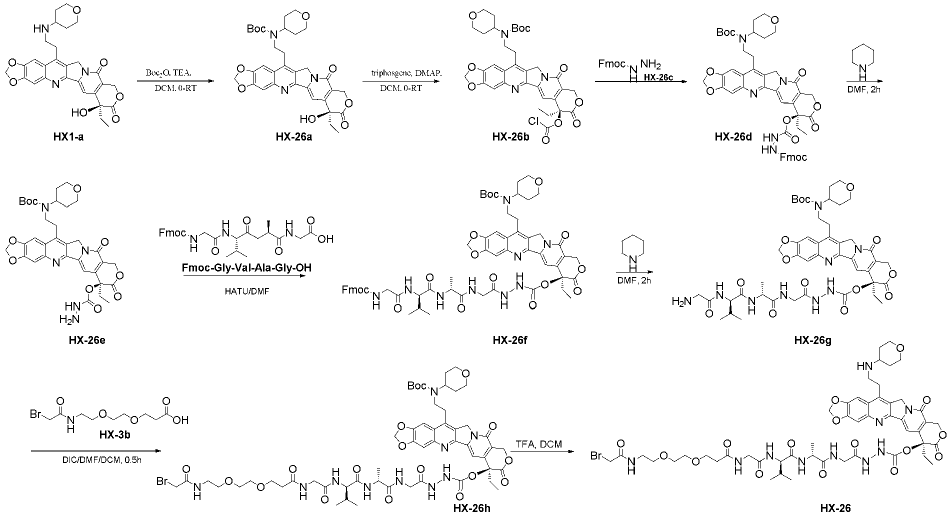

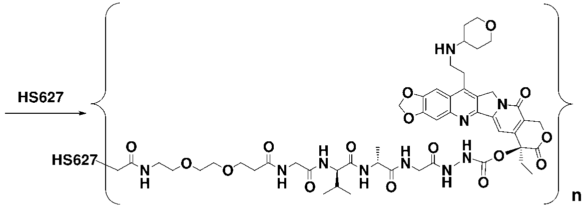

In a 50 mL single-neck flask, **HX-1a** (200 mg, 0.385 mmol) and 3 mL of dichloromethane were added. Then, Boc anhydride (92.4 mg, 0.423 mmol) and TEA (85.7 mg, 0.846 mmol) were added in sequence. The mixture was stirred to react at room temperature for 6 h. The solvent was removed under reduced pressure, and the residue was purified by silica gel column chromatography to afford **HX-26a** as a pale yellow solid. (223.5mg, yield 93.7%); LCMS: (M+1)⁺ 620.26 (theoretical value: 619.67).

In a 50 mL single-neck flask, **HX-26a** (85 mg, 0.137 mmol), DMAP (83.8 mg, 0.686 mmol) and 2 mL of dichloromethane were added in sequence. The mixture was stirred to react at 0°C for 5 minutes, then 2 mL of dichloromethane and triphosgene (32.6 mg, 0.11 mmol) were added. The mixture was allowed to react at room temperature for 2 h to afford a clear yellow solution of **HX-26b,** which was used directly in the next step.

To the clear yellow solution of **HX-26b** was directly added **HX-26c** (69.8 mg, 0.274 mmol). The mixture was stirred to react at room temperature for 3 h, then purified by silica gel column chromatography to afford **HX-26d** as a pale yellow solid (118.8 mg, yield 96.2%). LCMS: (M+1)⁺ 900.34 (theoretical value: 899.95).

In a 10 mL single-neck flask, **HX-26d** (100 mg, 0.11 mmol) was added and dissolved in 1 mL of DMF. Then, piperidine (94.6 mg, 1.1 mmol) was added. the mixture was reacted at room temperature for 0.5 h. The reaction solution was added dropwise to 20 mL of methyl tert-butyl ether, centrifuged, and the supernatant was removed. After drying, **HX-26e** was afforded. (70.5 mg, yield 93.2%); LCMS: (M+1)⁺ 678.27 (theoretical value: 677.71).

**HX-26e** (70 mg, 0.103 mmol) was taken and dissolved in 2 mL of DMF. HATU (47.2 mg, 0.124 mmol), DIPEA (16.02 mg, 0.124 mmol), and Fmoc-Gly-Val-Ala-Gly-OH (54.08 mg, 0.103 mmol) were added. The mixture was reacted at room temperature for 2 h, then diluted with 200 mL of DCM, washed with saturated sodium chloride solution, dried over anhydrous sodium sulfate, and purified by silica gel column chromatography to afford **HX-26f** as a yellow solid. (101.3 mg, yield 82.9%); LCMS: (M+1)⁺ 1082.49 (theoretical value: 1183.28).

In a 10 mL single-neck flask, **HX-26f** (101 mg, 0.085 mmol) was added, then dissolved with 1 mL of DMF, then piperidine (72.7 mg, 0.85 mmol) was added. The mixture was reacted at room temperature for 0.5 h. The reaction solution was added dropwise to 20 mL of methyl tert-butyl ether, centrifuged, and the supernatant was removed. After drying, **HX-26g** was afforded. (79.8 mg, yield 97.3%); LCMS: (M+1)⁺ 961.04 (theoretical value: 960.42).

In a 10 mL single-neck flask, DCM (2 mL), **HX-26g** (70 mg, 0.073 mmol), **HX-3b** (26 mg, 0.087 mmol) and DIC (11.02 mg, 0.087 mmol) were added in sequence. After stirring to react at room temperature for 30 min, HPLC indicated complete consumption of the starting materials, to afford a clear yellow solution of **HX-26.** Then 200 microlitre trifluoroacetic acid was added. The mixture was stirred to react at room temperature for 30 min. The reaction solution was injected into a 25 g C18 pre-column (pre-equilibrated with acetonitrile, then with water, the water phase contains 0.1% TFA), then eluted via medium-pressure reverse-phase C18 chromatography (gradient: 5% to 40% acetonitrile in water over 30min). The product was lyophilized to afford **HX-26** as a yellow solid (37.5 mg, yield 45%); LCMS: (M+1)⁺ 1142.03 (theoretical value: 1140.38).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-26** (0.91mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 17** (3.2 mg/mL, 2 mL).

UV-HPLC calculated average value: n=7.3.

### Example 18: 20-O-(IP140B-AcPEG2-Gly-Val-Ala-Gly-NHNHC)-7-(N-(tetrahydropyran-4-yl)amino)ethyl-10,11-methylenedioxycamptothecin (18)

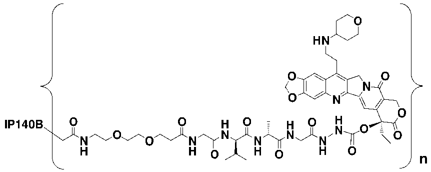

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-26** (0.91 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH5.7, to afford the antibody-drug conjugate **ADC 18** (3.3 mg/mL, 2 mL).

UV-HPLC calculated average value: n=7.5.

### Example 19: 20-O-(HS627-Ac-PEG4-daP-NHNHC)-7-(N-(tetrahydropyran-4-yl)amino)ethyl-10,11-methylenedioxycamptothecin (19)

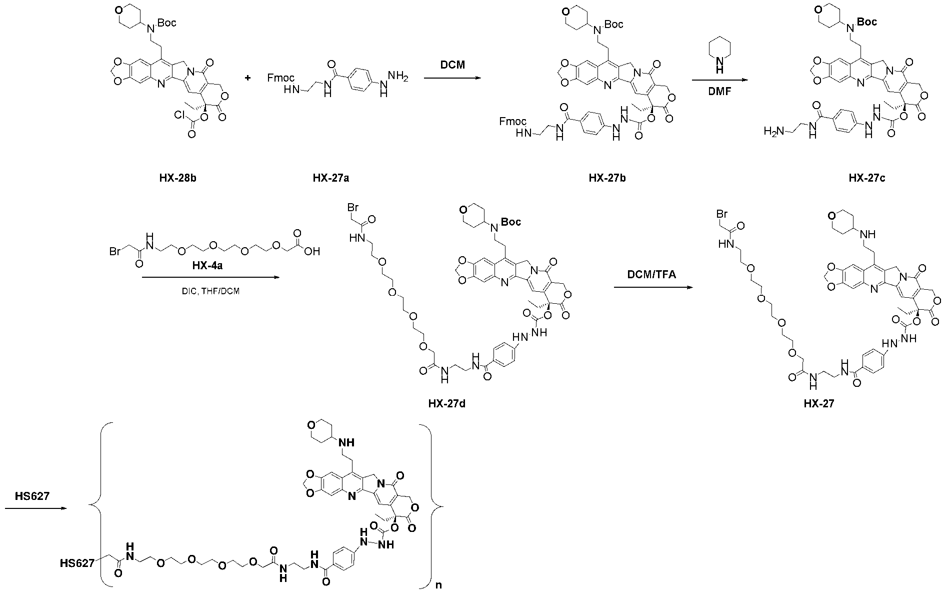

To the clear yellow solution of **HX-28b** prepared as described above was added **HX-27a** (80.8 mg, 0.274 mmol). The mixture was stirred to react at room temperature for 3 h, then purified by silica gel column chromatography to afford **HX-27b** as a pale yellow solid. (140.3 mg, yield 96.3%); LCMS: (M+1)⁺ 1062.15 (theoretical value: 1061.42).

In a 10 mL single-neck flask, **HX-27b** (140.3 mg, 0.132 mmol) was added and dissolved with 1 mL of DMF. Then, piperidine (112.6 mg, 1.32 mmol) was added, and the mixture was reacted at room temperature for 0.5 h. The reaction solution was added dropwise to 20 mL of methyl tert-butyl ether, centrifuged, and the supernatant was removed. After drying, **HX-27c** was afforded. (89.5 mg, yield 80.6%); LCMS: (M+1)⁺840.35 (theoretical value: 839.90).

In a 10 mL single-neck flask, DCM (2 mL), **HX-27c** (70 mg, 0.083 mmol), **HX-4a** (38.6 mg, 0.1 mmol), and DIC (12.6 mg, 0.1 mmol) were added in sequence. The mixture was stirred to react at room temperature for 30 min to afford a clear yellow solution of **HX-27d.** Then, 200 microlitre trifluoroacetic acid was added, and the mixture was stirred to react at room temperature for 30 min. The reaction solution was injected into a 25 g C18 pre-column (pre-equilibrated with acetonitrile, then with water, the water phase contains 0.1% TFA) , then eluted via medium-pressure reverse-phase C18 chromatography (gradient: 5% to 40% acetonitrile in water over 30min). The product was lyophilized to afford **HX-7** as a yellow solid (43.9 mg, yield 47.9%); LCMS: (M+1)⁺ 1093.98 (calculated value: 1092.38).

HS627 antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-27** (0.87mg, 0.8mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose and 0.2 g/L polysorbate 20, to afford the antibody-drug conjugate **ADC 19** (3.1 mg/mL, 2 mL).

UV-HPLC calculated average value: n=7.5.

### Example 20: 20-O-(IP140B-Ac-PEG4-daP-NHNHC)-7-(N-(tetrahydropyran-4-yl)amino)ethyl-10,11-methylenedioxycamptothecin (20)

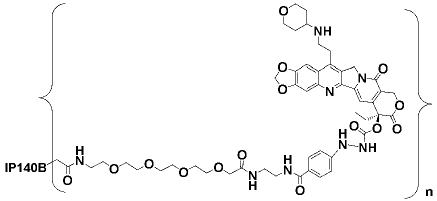

IP140B antibody (20.0mg/mL, 10mg, 0.066mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.04 mL) was added. The mixture was rotated to react at 25°C for 90 min.

Compound **HX-27** (0.87 mg, 0.8 mmol) was dissolved in 0.09 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250mM sorbitol, 0.02% Tween 80, pH5.7, to afford the antibody-drug conjugate **ADC 20** (3.3 mg/mL, 2 mL).

UV-HPLC calculated average value: n=7.4.

### Example 21: 7-(N-(HS627-AcPEG2-Gly-Lys-PABC), N-(tetrahydropyran-4-yl)amino)ethyl-10,11-methylenedioxycamptothecin (21)

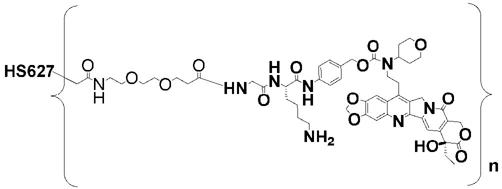

S627 antibody (20.0 mg/mL, 10 mg, 0.066 mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.016 mL) was added. The mixture was rotated to react at 25°C for 90 min.

Compound **HX-3** (0.42 mg, 0.367 mmol) was dissolved in 0.04 mL of DMA and added to the above solution system. After mixing well, the mixture was rotated to react at room temperature for 2 h. After the reaction was completed, the buffer was exchanged using a NAP-5 gel column (Cytiva) into 20 mM L-histidine acetate buffer, pH 6.0, 120 mM sucrose, 0.2g/L polysorbate 20, to afford the antibody-drug conjugate **ADC-21** (3 mg/mL, 2 mL).

UV-HPLC calculated average value: n=4.05

### Example 22: 7-(N-(IP140B-AcPEG2-Gly-Lys-PABC), N-(tetrahydropyran-4-yl)amino)ethyl-10,11-methylenedioxycamptothecin (22)

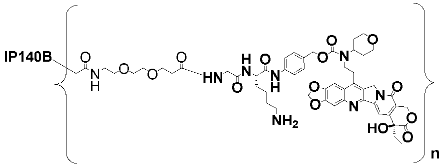

IP140B antibody (20.0 mg/mL, 10 mg, 0.066 mmol) was taken and adjusted pH to 7.2 with 1 M Na₂HPO₄ solution. Then, a solution of 0.1 M disodium ethylenediaminetetraacetate (25 µL) was added, then a solution of prepared TCEP·HCl (tris(2-carboxyethyl)phosphine hydrochloride) (10 mM, 0.016 mL) was added. The mixture was rotated to react at 25°C for 90 min.

Compound **HX-3** (0.42 mg, 0.367 mmol) was dissolved in 0.04 mL of DMA and added to the above solution system. After mixing well, the mixture was rotated to react at room temperature for 2 h. After the reaction was completed, the buffer was exchanged using a NAP-5 gel column (Cytiva) into 20 mM histidine solution, 250 mM sorbitol, 0.02% Tween 80, pH 5.7, to afford the antibody-drug conjugate **ADC-22** (3.1 mg/mL, 2mL).
UV-HPLC calculated average value: n=3.95

### Assay 1: In vitro inhibitory activity of ADC against tumor cell growth

Method for *in vitro* inhibitory activity assay of ADC: Human esophageal cancer cells OE-33, lung cancer cells NCI-H1975, and breast cancer cells MDA-MB-231 used for activity assays were respectively cultured in RPMI1640 (Cellmax), RPMI1640 (Cellmax), and DMEM (Cellmax) media containing 10% fetal bovine serum (FBS, Cellmax) to the exponential growth phase. After trypsinization, the cells were centrifuged, and the supernatant was discarded. The cells were resuspended with media and diluted to 3x10⁴ cells/mL, 0.5x10⁴ cells/mL and 1.5x10⁴ cells/mL respectively. Then, 100 µL of each cell suspension was added to a 96-well cell culture plate, and the plate was returned to an incubator at 37°C with 5% CO₂ for overnight culture. The next day, the ADC to be tested was diluted to 2000 nM, 400 nM, 80 nM, 16 nM, 3.2 nM, 0.64 nM, 0.128 nM, and 0.026 nM using culture medium. The diluted ADC was added to a 96-well cell culture plate at 100 µL per well, with 3 replicates set for each concentration. For the negative control without ADC and the blank control, 100 µL of culture medium was added to each well. After the sample addition was finished, the plate was returned to the incubator at 37°C with 5% CO₂ for further incubation for 6 days. Upon completion of incubation, the cell culture plate was removed, and the medium in the plate was aspirated using a pipette. 100 µL of medium containing 10% CCK-8 was added to each well, followed by incubation at 37°C for 3 h. After incubation, the plate was removed, protected from light, placed in a microplate reader, and the absorbance was measured at a test wavelength of 450 nm with a reference wavelength of 630 nm. Based on the absorbance values, the IC₅₀ (Table 1) was calculated using four-parameter regression in GraphPad. The corresponding ADC drug containing Dxd was used as a positive control drug, which has the following structure: 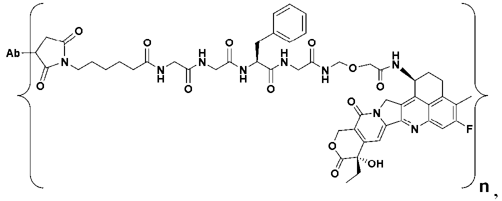 wherein, Ab is the IP140B antibody.

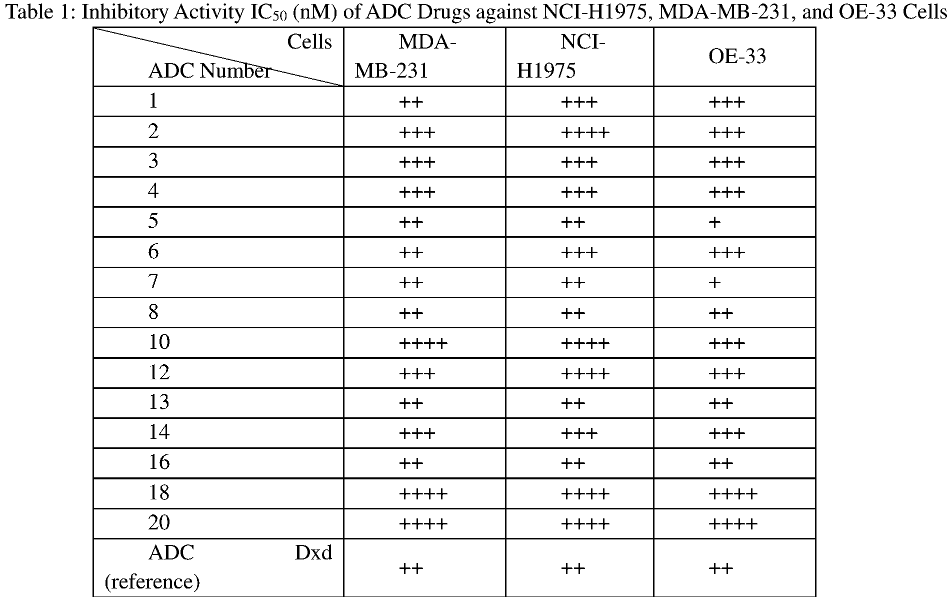

For the IC₅₀ values, wherein "++++" indicates IC₅₀ < 5 nM; "+++" indicates IC₅₀ of 5 nM-50 nM; "++" indicates IC₅₀ of 50 nM-500 nM; and "+" indicates IC₅₀ > 250 nM.

The ADC compounds of the examples in the present disclosure all have good inhibitory activity against NCI-H1975, MDA-MB-231, and OE-33 cells. Their inhibitory activity IC₅₀ against these cells are all lower than 250 nM. Some ADC compounds such as ADC2, 3, 4, 10, 12, 14, 18, and 20 even all have inhibitory activity IC₅₀ against these cancer cells lower than 50 nM. Some ADC compounds such as ADC4, 10, 18, and 20 even all have inhibitory activity IC₅₀ against these cancer cells lower than 10 nM.

### Assay 2: In vivo inhibitory activity of ADC against tumor growth

Method for *in vivo* inhibitory activity assay of ADC: Human lung cancer cells NCI-H292 and NCI-H1975 were cultured *in vitro* as a monolayer. When the cell saturation was 80%-90%, they were digested with trypsin-EDTA, centrifuged, and the supernatant was discarded. The cells were resuspended with PBS, and the cell suspension was adjusted to a suitable concentration. NCI-H292 and NCI-H1975 cells (2-10x10⁶ cells/0.1mL) were subcutaneously inoculated into BALB/c nude mice. The animals and the growth of transplanted tumors were regularly observed. When the tumor volume was grown to about 100-200 mm³, random grouping was performed according to tumor volume and body weight, namely the vehicle control group (normal saline) and the ADC administration group (dissolved in normal saline), with 6 animals per group. Administration was performed by intravenous injection at a dosing frequency of Q4D for a total of 2 times (the first administration was recorded as Day 1, and the second administration was on Day 5). Experimental grouping and dosing settings were shown in Table 2. Tumor major diameter a (mm), minor diameter b (mm), and mice body weight were measured with a vernier caliper twice a week. Tumor volume (V) was calculated according to the following formula: V = 1/2xaxb² (mm³), wherein a and b represents the tumor length and width, respectively. Growth curves were plotted, and finally, tumors were excised and weighed. Statistical analysis was performed using GraphPad Prism software based on tumor volume and body weight data of tumor-bearing mice at the end of the trial to afford tumor inhibition results. In the figures, "\" in the control group indicates no results, with tumor status unmeasured; the experimental group had no corresponding tumors. "Tumor volume reduced to 0" and "tumor disappeared to 0" indicates no tumor residue was found in the corresponding dissected animals.

**Table 2: Mouse Grouping and Administration Settings**

| Group | Cell Model | Group/Drug Injected | Administration Dose | Tumor Growth Curves | Photographs of Tumors after Dissection |
|---|---|---|---|---|---|
| 1 | NCI-H292 | IP1408 antibody | 20mg/kg weight | Figure 1 | Figure 2 |
| | | Vehicle control group | - | | |
| | | ADC Dxd | 3mg/kg weight | | |
| | | | 6mg/kg weight | | |
| | | | 10mg/kg weight | | |
| | | ADC 4 | 3mg/kg weight | | |
| | | | 6mg/kg weight | | |
| | | | 10mg/kg weight | | |
| 2 | NCI-H1975 | Vehicle control group | - | Figure 3 | Figure 4 |
| | | ADC Dxd | 10mg/kg weight | | |
| | | ADC 4 | 1mg/kg weight | | |
| | | | 3mg/kg weight | | |
| | | | 10mg/kg weight | | |
| 3 | NCI-H1975 | Vehicle control group | - | Figure 5 | Figure 6 |
| | | ADC Dxd | 3mg/kg weight | | |
| | | | 10mg/kg weight | | |
| | | ADC 6 | 3mg/kg weight | | |
| | | | 10mg/kg weight | | |
| | | ADC 8 | 10mg/kg weight | | |
| | | ADC 22 | 3mg/kg weight | | |
| | | | 10mg/kg weight | | |
| | | Vehicle control group | - | | |
| | | ADC Dxd | 3mg/kg weight | | |
| | | | 10mg/kg weight | | |
| 4 | NCI-H1975 | ADC 14 | 3mg/kg weight | Figure 7 | Figure 8 |
| | | | 10mg/kg weight | | |
| | | ADC 16 | 3mg/kg weight | | |
| | | | 10mg/kg weight | | |
| | | ADC 12 | 3mg/kg weight | | |
| | | | 10mg/kg weight | | |

The above descriptions of specific exemplary embodiments of the present disclosure are for purposes of illustration and exemplification. These descriptions are not intended to limit the present disclosure to the precise forms disclosed, and it is apparent that many changes and modifications can be made in light of the above teachings. The purpose of selecting and describing exemplary embodiments is to explain the specific principles of the present disclosure and practical applications thereof, thereby enabling those skilled in the art to implement and utilize various exemplary embodiments of the present disclosure, along with various selections and modifications. The scope of the present disclosure is intended to be defined by the claims and equivalents thereof.

## Claims

1. An antibody-drug conjugate represented by Formula (I), 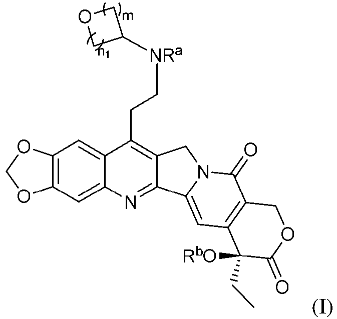 in the Formula,
m, n₁ are each independently selected from 1 or 2;
R^{a} is selected from hydrogen and -L¹-NH-L^{p}-Z-Ab, R^{b} is selected from hydrogen, -L²-NH-L^{p}-Z-Ab and -L³-NH-Z-Ab, provided that R^{a}, R^{b} are not both hydrogen;
wherein,
L¹ is selected from -C(=O)-L¹¹-L¹²-L¹³-;
L¹¹, L¹², L¹³ are each independently selected from -O-, C₁-C₃ alkylene and phenyl;
L² is selected from -C(=O)-NH-L²¹-CHR¹-;
L²¹ is selected from -C(=O)- and -NH-C(=O)-;
R¹ is selected from hydrogen, deuterium, C₁-C₆ alkyl and phenyl-substituted C₁-C₆ alkyl;
L³ is selected from -C(=O)-NR²-NR³-L³¹-L³²-;
R², R³ are each independently selected from hydrogen, deuterium and C₁-C₆ alkyl;
L³¹ is selected from -C(=O), -(C₁-C₃ alkylene)-C(=O), -5-8 membered aryl-C(=O)-, -5-8 membered azaheteroaryl-C(=O)- and -O-(C₁-C₃ alkylene)-C(=O)-;
L³² is selected from -NH-(C₁-C₃ alkylene), -N(CH₃)₂-(C₁-C₃ alkylene) and -NH-(C₁-C₃ alkylene)-NH-;
L^{P} is a peptide residue consisting of 2 to 7 amino acids;
Z is selected from -L^{z}-L^{j}-, wherein, L^{z} is selected from -C(=O)-C₁-C₈ alkylene, -C(=O)-CH₂O-(CH₂CH₂O)₂₋₅-CH₂CH₂NH-, -C(=O)-(CH₂CH₂O)₂₋₆-CH₂CH₂- and -C(=O)-(CH₂CH₂O)₂₋₆-CH₂CH₂NH-, L^{j} is a linker that can conjugate to an antibody;
Ab is an antibody.

2. The antibody-drug conjugate according to claim 1, wherein, the structure represented by 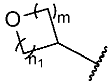 is selected from 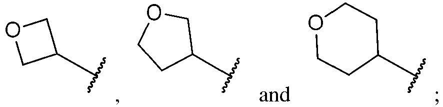 preferably, L¹¹, L¹², L¹³ are each independently selected from -O-, methylene and phenyl;
preferably, L¹¹-L¹²-L¹³ is selected from -O-CH₂-phenyl- and -CH₂-O-CH₂-;
preferably, L¹ is selected from -C(=O)-O-CH₂-phenyl.

3. The antibody-drug conjugate according to claim 1 or 2, wherein, R¹ is selected from hydrogen, deuterium, C₁-C₆ alkyl and phenyl-substituted C₁-C₆ alkyl;
preferably, R¹ is selected from hydrogen, deuterium, methyl, isopropyl, isobutyl and phenylmethyl;
preferably, R¹ is hydrogen;
preferably, L² is selected from -C(=O)-NH-C(=O)-CH₂- and -CH₂-NH-NH-C(=O)-CH₂-;
preferably, L² is -CH₂-NH-NH-C(=O)-CH₂-.

4. The antibody-drug conjugate according to any one of claims 1-3, wherein, R², R³ are each independently selected from hydrogen, deuterium and C₁-C₃ alkyl;
preferably, R², R³ are each independently selected from hydrogen, deuterium and methyl;
preferably, R² is hydrogen, R³ is hydrogen;
preferably, L³¹ is selected from -C(=O), -methylene-C(=O), -phenyl-C(=O)-, -pyridyl-C(=O)- and -O-CH₂-C(=O)-;
preferably, L³² is selected from -NH-CH₂-CH₂-, -N(CH₃)₂-CH₂-CH₂- and -NH-CH₂-NH-;
preferably, L³ is -C(=O)-NH-NH-phenyl-C(=O)-NH-CH₂-CH₂-.

5. The antibody-drug conjugate according to any one of claims 1-4, wherein, the amino acid is selected from phenylalanine (Phe), glycine (Gly), valine (Val), alanine (Ala), leucine (Leu), lysine (Lys), citrulline (Cit), and asparagine (Asn);
preferably, L^{p} is selected from peptide residues consisting of 2-5 amino acids selected from phenylalanine (Phe), glycine (Gly), valine (Val), alanine (Ala), leucine (Leu), lysine (Lys), citrulline (Cit), and asparagine (Asn);
preferably, L^{p} is selected from peptide residues consisting of 2 or 3 amino acids selected from glycine (Gly), valine (Val), alanine (Ala), lysine (Lys), and citrulline (Cit);
preferably, L^{p} is selected from Val-Cit, Val-Ala, Gly-Val-Ala, Gly-Val-Ala-Gly, Gly-Lys, Gly-Gly-Lys, Gly-Gly-Lys-Gly, Val-Ala, Ala-Ala-Asn, Gly-Leu, Gly-Gly-Leu, Gly-Gly-Leu-Gly, Gly-Phe, Gly-Gly-Phe and Gly-Gly-Phe-Gly;
preferably, L^{p} is selected from 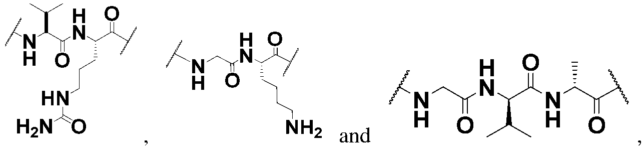 wherein the carbonyl terminus is linked to -NH- and the other terminus is linked to Z;
preferably, L^{j} is selected from 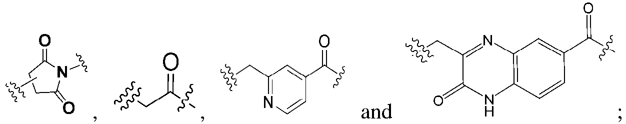 the position represented by " 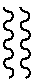 " is linked to the antibody, and the position represented by " 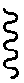 " is linked to the L^{Z} group;
preferably, L^{z} is selected from -C(=O)-C₁-C₈ alkylene, -C(=O)-CH₂O-(CH₂CH₂O)₂₋₅-CH₂CH₂NH-, -C(=O)-(CH₂CH₂O)₂₋₆-CH₂CH₂- and -C(=O)-(CH₂CH₂O)₂₋₆-CH₂CH₂NH-;
preferably, L^{z} is selected from -C(=O)-C₁-C₆ alkylene, -C(=O)-CH₂O-(CH₂CH₂O)₂₋₃-CH₂CH₂NH-, -C(=O)-(CH₂CH₂O)₂₋₄-CH₂CH₂- and -C(=O)-(CH₂CH₂O)₂₋₄-CH₂CH₂NH-;
preferably, L^{z} is selected from -C(=O)-(CH₂)₅-, -C(=O)-CH₂O-(CH₂CH₂O)₃-CH₂CH₂NH-, -C(=O)-(CH₂CH₂O)₂-CH₂CH₂-, -C(=O)-(CH₂CH₂O)₂-CH₂CH₂NH- and -C(=O)-(CH₂CH₂O)₄-CH₂CH₂NH-;
preferably, Z is selected from 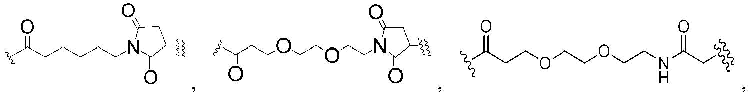 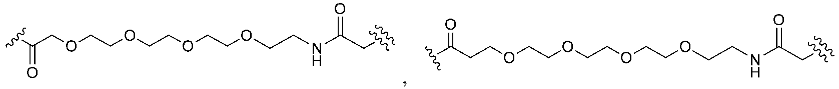 and 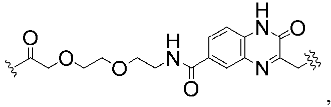 the position represented by " 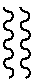 " is linked to the antibody, and the position represented by " 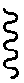 " is linked to the L^{p} group or L³ group.

6. The antibody-drug conjugate according to any one of claims 1-5, wherein, the antibody is an antibody against tumor-associated antigen;
preferably, the antibody against tumor-associated antigen is selected from anti-Her2 antibody, anti-Trop2 antibody, anti-B7H3 antibody, anti-5T4 antibody, anti-Nectin-4 antibody, anti-CD20 antibody, and anti-ROR1 antibody.

7. The antibody-drug conjugate according to any one of claims 1-6, the antibody-drug conjugate represented by Formula (I) has a structure represented by Formula (I-1), Formula (I-2) or Formula (I-3), 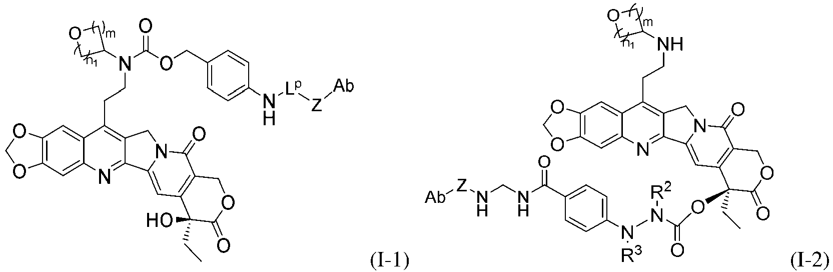 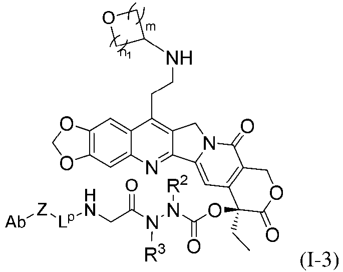 in the Formula, R², R³, m, n₁, L^{p}, Z, Ab are defined the same as the compound of Formula (I) at each occurrence.

8. The following antibody-drug conjugate: 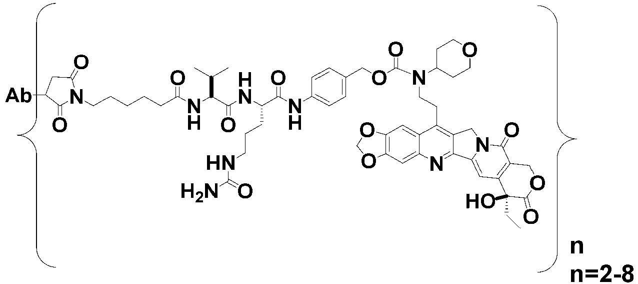 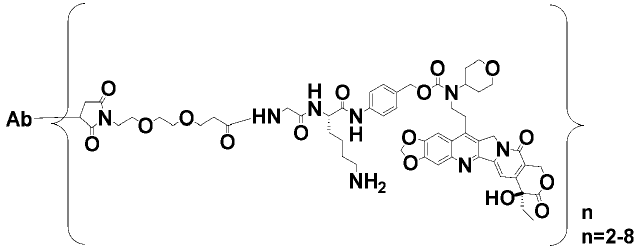 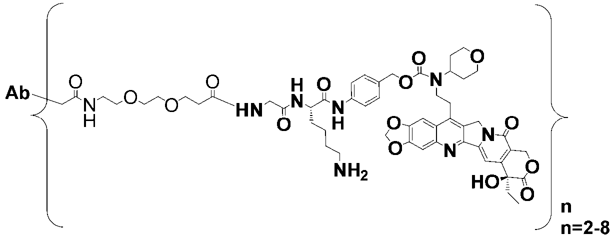 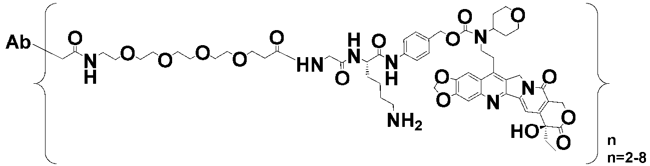 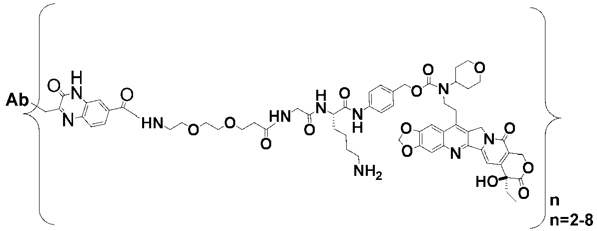 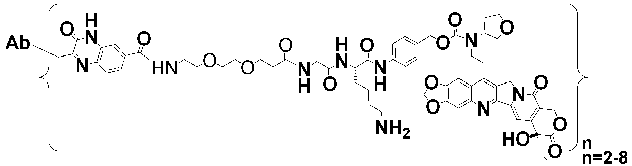 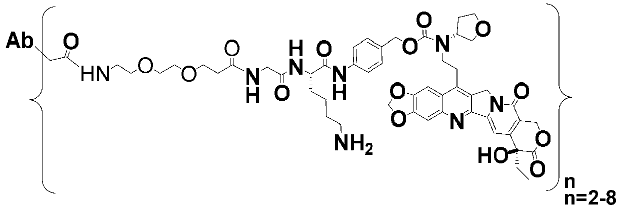 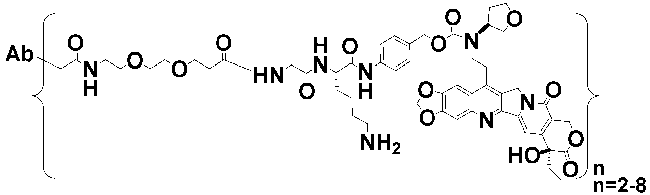 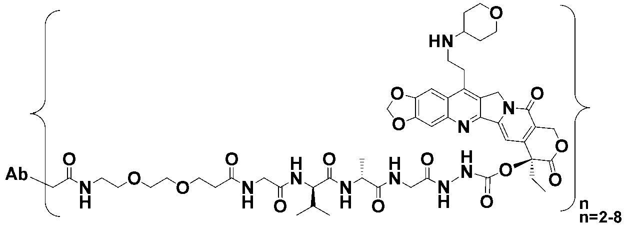 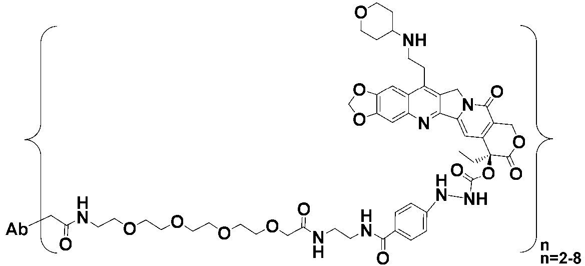
wherein, Ab is defined the same as the compound of Formula (I);
preferably, Ab is the HS627 antibody or IP140B antibody, the heavy chain amino acid sequence of HS627 is as set forth in SEQ ID NO: 1, and the light chain amino acid sequence thereof is as set forth in SEQ ID NO: 2; the heavy chain amino acid sequence of the IP140B antibody is as set forth in SEQ ID NO: 3, and the light chain amino acid sequence thereof is as set forth in SEQ ID NO: 4.

9. A pharmaceutical composition comprising the antibody-drug conjugate according to any one of claims 1-8 and a pharmaceutically acceptable carrier.

10. Use of the antibody-drug conjugate according to any one of claims 1-8 or the pharmaceutical composition according to claim 9 in the manufacture of an anti-tumor medicament.
preferably, the tumor is selected from a solid tumor, more preferably selected from esophageal cancer, lung cancer, or breast cancer.

11. A method for treating a tumor disease, comprising the step of administering to a patient in need thereof the antibody-drug conjugate according to any one of claims 1-8 or the pharmaceutical composition according to claim 9.
